# EUROPEAN PATENT SPECIFICATION

(11) **EP 2 209 432 B1**
(45) Date of publication and mention of the grant of the patent: **25.12.2013**
(21) Application number: 08843605.0
(22) Date of filing: 31.10.2008
(51) Int. Cl.: A61B 17/00, A61B 18/00, A61B 18/14, A61N 1/32

(54) **Energy delivery device comprising an electrode cartridge**
Gerät zur Energielieferung mit einem Elektrodeneinsatz
Dispositif pour fournir de l'énergie comprenant une cartouche d'électrodes

(30) Priority: 31.10.2007 US 984303 P; 01.02.2008 US 24925; 12.03.2008 US 35936 P
(43) Date of publication of application: 28.07.2010
(73) Proprietor: Syneron Medical Ltd., 20692 Yoqneam Illit (IL)
(72) Inventor: MCGILL, Scott, San Ramon California 94582 (US); MEHTA, Bankim H., San Ramon California 94582 (US); KNOPP, Peter G., Pleasanton California 94566 (US)
(74) Representative: Price, Nigel John King
(86) International application number: PCT/US2008/082061
(87) International publication number: WO 2009/059186

(56) References cited:
- WO-A1-01/78832
- US-A1- 2002 120 260
- US-A1- 2005 199 605
- US-A1- 2005 222 565
- US-A1- 2007 082 040
- US-A1- 2007 106 143
- US-A1- 2007 112 343
- US-A1- 2007 232 984

## Description

### BACKGROUND OF THE INVENTION

The systems discussed herein treat tissue in the human body. In a particular variation, systems described below treat cosmetic conditions affecting the skin of various body parts, including face, neck, and other areas traditionally prone to wrinkling, lines, sagging and other distortions of the skin.

Exposure of the skin to environmental forces can, over time, cause the skin to sag, wrinkle, form lines, or develop other undesirable distortions. Even normal contraction of facial and neck muscles, e.g. by frowning or squinting, can also over time form furrows or bands in the face and neck region. These and other effects of the normal aging process can present an aesthetically unpleasing cosmetic appearance.

Accordingly, there is a well known demand for cosmetic procedures to reduce the visible effects of such skin distortions. There remains a large demand for 'tightening' skin to remove sags and wrinkles, especially in the regions of the face and neck.

One method surgically resurfaces facial skin by ablating the outer layer of the skin (from 200 µm to 600 µm), using laser or chemicals. In time, a new skin surface develops. The laser and chemicals used to resurface the skin also irritate or heat the collagen tissue present in the dermis. When irritated or heated in prescribed ways, the collagen tissue partially dissociates and, in doing so, shrinks. The shrinkage of collagen also leads to a desirable 'tightened' look. Still, laser or chemical resurfacing leads to prolonged redness of the skin, infection risk, increased or decreased pigmentation, and scarring.

Lax et al. U.S. Pat. No. 5,458,596 describes the use of radio frequency energy to shrink collagen tissue. This cosmetically beneficial effect can be achieved in facial and neck areas of the body in a minimally intrusive manner, without requiring the surgical removal of the outer layers of skin and the attendant problems just listed.

Utely et al. U.S. Pat. No. 6,277,116 also teaches a system for shrinking collagen for cosmetically beneficial purposes by using an electrode array configuration.

However, areas of improvement remain with the previously known systems. In one example, fabrication of an electrode array may cause undesired cross-current paths forming between adjacent electrodes resulting in an increase in the amount of energy applied to tissue.

In another example, when applying the array to tissue, the medical practitioner experiences a "bed-of-nails". In other words, the number of electrodes and their configuration in the array effectively increases the total surface area of the electrode array. The increase in effective surface area then requires the medical practitioner to apply a greater force to the electrode array in order to penetrate tissue. Such a drawback may create collateral damage as one or more electrodes may be placed too far within the skin. Additionally, the patient may experience the excessive force as the medical practitioner increases the applied force to insert the array within tissue.

Thermage, Inc. of Hayward California also holds patents and sells devices for systems for capacitive coupling of electrodes to deliver a controlled amount of radiofrequency energy. This controlled delivery of RF energy creates an electric field through the epidermis that generates 'resistive heating' in the skin to produce cosmetic effects while simultaneously attempting to cool the epidermis with a second energy source to prevent external burning of the epidermis.

In such systems that treat in a non-invasive manner, generation of energy to produce a result at the dermis results in unwanted energy passing to the epidermis. Accordingly, excessive energy production creates the risk of unwanted collateral damage to the skin.

In view of the above, there remains a need for an improved energy delivery system. Such systems may be applied to create an improved electrode array delivery system for cosmetic treatment of tissue. In particular, such an electrode array may provide deep uniform heating by applying energy to tissue below the epidermis to cause deep structures in the skin to immediately tighten. Over time, new and remodeled collagen may further produce a tightening of the skin, resulting in a desirable visual appearance at the skin's surface. Such systems can also provide features that increase the likelihood that the energy treatment will be applied to the desired target region. Moreover, devices and systems having disposable or replaceable energy transfer elements provide systems that offer flexibility in delivering customized treatment based on the intended target tissue.

Moreover, the features and principles used to improve these energy delivery systems can be applied to other areas, whether cosmetic applications outside of reduction of skin distortions or other medical applications.

In one variation, devices are described to enable more reliable and less expensive fabrication of electrode assemblies. More specifically, the mounting of needle electrodes directly to printed circuit boards can increase the consistency and quality of the device and reduce the labor component.

There is a great deal of labor/assembly steps necessary for manufacturing a multi-electrode device. These steps include soldering electrical leads to the electrodes and mounting the electrodes on a carrier/base. These steps require higher manual dexterity as the electrode size decreases. Along with those challenges the error rate increases as the task becomes more difficult. This has a significant impact: when the 10th or 20th electrode in an assembly is bad then the entire assembly cannot be used. In one variation, a Printed Circuit Board (PCB) is fabricated automatically and very inexpensively that contains as part of the standard fabrication process lead lined grooves which are configured to hold the electrodes. The electrodes are placed in the grooves and easily hand soldered or machine wave soldered or soldered using another automated soldering technique to the board. This provides both the electrical and mechanical functions in one automated and inexpensive step.

US 2007/232984 A1 discloses a hand-held electrical stimulation device for providing waveforms that are effective in promoting wound healing, improving circulation, stimulating peripheral nerves, administering pharmaceutical compounds via electrophoresis and electroporating DNA into tissue. The apparatus is shaped similarly to a hand gun and comprises two main components: a disposal electrode cartridge and a hand-held stimulation system. The disposable electrode cartridge is adapted to snap into and out of the housing of the stimulation system using a snap lock system. The electrode cartridge contains a plurality of electrodes that remain covered until the system is engaged. To perform electroporation, a linear actuator is used to inject DNA into the target tissue. Once activated, the linear actuator extends to depress the plunger on a syringe containing DNA solution, thus injecting the solution into tissue. After the syringe is fully injected and any electrical fields have been applied, the linear actuator retracts such that it can be employed for the next injection.

### SUMMARY OF THE INVENTION

According to a first aspect of the present invention there is provided the energy delivery device of claim 1.

According to a second aspect of the present invention there is provided a kit for applying therapeutic energy to tissue, the kit comprising the energy delivery device of the above first aspect of the present invention and at least one further said cartridge body, each further said cartridge body being capable of being removably coupled to the device body on the cartridge receiving surface, and
where the electrode assembly on each respective cartridge body has a different configuration.

### BRIEF DESCRIPTION OF THE DRAWINGS

FIG. 1 shows a representative sectional view of skin and underlying subcutaneous tissue;

FIG. 2A shows a sample variation of a system according to the principles of the invention;

FIG. 2B illustrates a partial view of a working end of a treatment unit engaging tissue such that the array enters the tissue;

FIG. 3A illustrates a perspective view of a variation of a cartridge body for use with the present system;

FIG. 3B shows a cross sectional view of the cartridge body of FIG. 3A;

FIGS. 3C to 3E show a cross sectional view of a cartridge body where the electrodes are offset to prevent the formation of a continuous line of insertion marks in the tissue;

FIG. 4A illustrates a cross sectional view of a variation of a treatment unit.

FIG. 4B shows a partial perspective cross sectional view of the treatment unit of FIG. 4A;

FIG. 4C shows a side cross sectional view of a working end of a treatment unit and cartridge coupled together;

FIGS. 4D and 4E illustrates a variation of multiple conductive leads on a connection board for selective coupling to multiple conductive leads on an electrode;

FIG. 5 shows a graph representing pulsed energy delivery and temperature measurements between pulses of energy;

FIGS. 6A to 6B show variations of introducer members that assist in placing electrodes within tissue;

FIG. 7A shows an additional variation of a device having an array of electrodes in a removable cartridge adjacent to a tissue engaging surface;

FIG. 7B shows a magnified view of the electrodes and tissue engaging surface of the device of FIG. 7A;

FIG. 7C shows an example of an electrode entering tissue at an oblique angle adjacent to a tissue engaging surface;

FIG. 8 shows another example of an electrode entering tissue at an oblique angle underneath a skin anomaly;

FIG. 9A to 9C show cooling surfaces adjacent to the electrodes;

FIGS. 10A-10D illustrate variations of electrodes having varying resistance or impedance along the length of the electrode; and

FIGS. 11A to 11B show an example of an array of electrodes where any number of pairs of electrodes can be triggered to apply therapeutic energy to tissue.

Figure 12A is a bottom view of a printed circuit board.

Figure 12B is a top view of the board shown in Figure 12A.

Figure 12C is a side view of the board shown in Figures 12A and 12B.

Figure 13A is a bottom view of the board shown in Figure 12A with the electrodes attached.

Figure 13B is a side view of the board shown in Figure 13A.

Figure 14 is a perspective view of the board shown in Figures 13A and 13B with an electrical connector attached..

Figure 15 is a perspective view of an alternated version of the printed circuit board shown in Figures 12A-12C.

Figures 16A and 16B are bottom and side views respectively of yet another alternative version.

Figure 17A is a bottom view of electrodes that are designed to be used as part of another version.

Figure 17B is a bottom view of another alternative version using the electrodes shown in Figure 17A.

Figure 18A is a bottom view of still another alternative printed circuit board portion.

Figure 18B is a cross-sectional view of the board shown in Figure 18A through line A-A.

Figure 19A is a bottom view of electrodes that are designed to be used as part of another version.

Figure 19B is a bottom view of another alternative version using the electrodes shown in Figure 19A.

Figure 20A is a bottom view of still another alternative printed circuit board portion.

Figure 20B is a cross-sectional view of the board shown in Figure 20A through line A-A.

Figure 21A is a bottom view of electrodes that are designed to be used as part of another version.

Figure 21B is a bottom view of another alternative version using the electrodes shown in Figure 21A.

### DESCRIPTION OF THE PREFERRED EMBODIMENT

The systems discussed herein treat tissue in the human body. In one variation, the systems treat cosmetic conditions affecting the skin of various body parts, including face, neck, and other areas traditionally prone to wrinkling, lines, sagging and other distortions of the skin. The systems described herein may also have application in other surgical fields apart from cosmetic applications.

The inventive device can also be used for treatment of skin anomalies such as warts (Verruca plana, Verruca vulgaris), sebaceous hyperplasia or acne (Acne vulgaris). Treatment of acne can be accomplished by the direct ablation of sebaceous glands or it can be accomplished by the delivery of thermal energy which will stimulate the body's immune system to eliminate the bacteria, Propionibacterium acnes, which is one of the causes of acne. The device can be used for the removal of unwanted hair (i.e., epilation) by applying energy or heat to permanently damage hair follicles thereby removing the skin's ability to grow hair. Such treatment may be applied on areas of facial skin as well as other areas of the body.

Other possible uses include pain management (both in the use of heat to reduce pain in muscle tissue and by directly ablating nociceptive pain fibers), stimulation of cellular healing cascade via heat, treatment of the superficial muscular aponeurotic system (SMAS), reproductive control by elevated heating of the testicles, and body modification such as piercing, scarification or tattoo removal

In addition to therapeutic surface treatments of the skin, the current invention can be targeted to the underlying layer of adipose tissue or fat for lipolysis or the breakdown of fat cells. Selecting electrodes having sufficient length to reach the subcutaneous fat layer allows for such electrodes to apply energy in the subcutaneous fat layer. Application of the energy can break down the fat cells in that layer allowing the body to absorb the resulting free fatty acids into the blood stream. Such a process can allow for contouring of the body surface for improved appearance. Naturally, such an approach can be used in the reduction of cellulite.

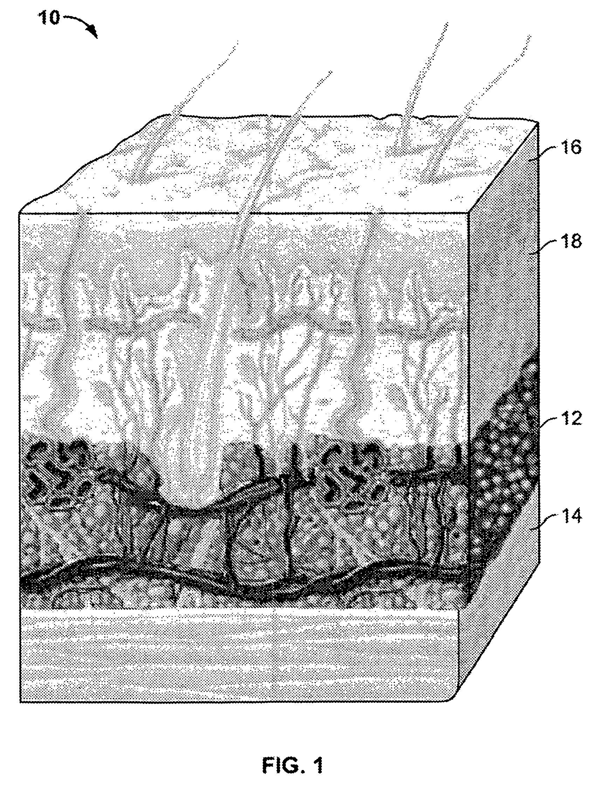

As FIG. 1 shows, the skin 10 covers subcutaneous tissue 12 and muscle tissue 14 of within the body. In the face and neck areas, the skin 10 measures about 2 mm in cross sectional depth.

The skin 10 includes an external, non-vascular covering called the epidermis 16. In the face and neck regions, the epidermis measures about 100 µm in cross sectional depth. The skin **10** also includes a dermis **18** layer that contains a layer of vascular tissue. In the face and neck regions, the dermis **18** measures about 1900 µm in cross sectional depth.

The dermis **18** includes a papillary (upper) layer and a reticular (lower) layer. Most of the dermis **18** comprises collagen fibers. However, the dermis also includes various hair bulbs, sweat ducts, sebaceous glands and other glands. The subcutaneous tissue **12** region below the dermis **18** contains fat deposits as well as vessels and other tissue.

In most cases, when applying cosmetic treatment to the skin for tightening or removal of wrinkles, it is desirable to deliver energy to the dermis layer rather than the epidermis, the subcutaneous tissue region **12** or the muscle **14** tissue. In fact, delivery of energy to the subcutaneous tissue region **12** or muscle **14** may produce pockets or other voids leading to further visible imperfections in the skin of a patient. Also, delivery of excessive energy to the epidermis can cause bums and/or scars leading to further visible imperfections.

The application of heat to the fibrous collagen structure in the dermis **18** causes the collagen to dissociate and contract along its length. It is believed that such disassociation and contraction occur when the collagen is heated to about 65 degrees C. The contraction of collagen tissue causes the dermis **18** to reduce in size, which has an observable tightening effect. As the collagen contracts, wrinkles, lines, and other distortions become less visible. As a result, the outward cosmetic appearance of the skin **10** improves. Furthermore, the eventual wound healing response may further cause additional collagen production. This latter effect may further serve to tighten and bulk up the skin **10.**

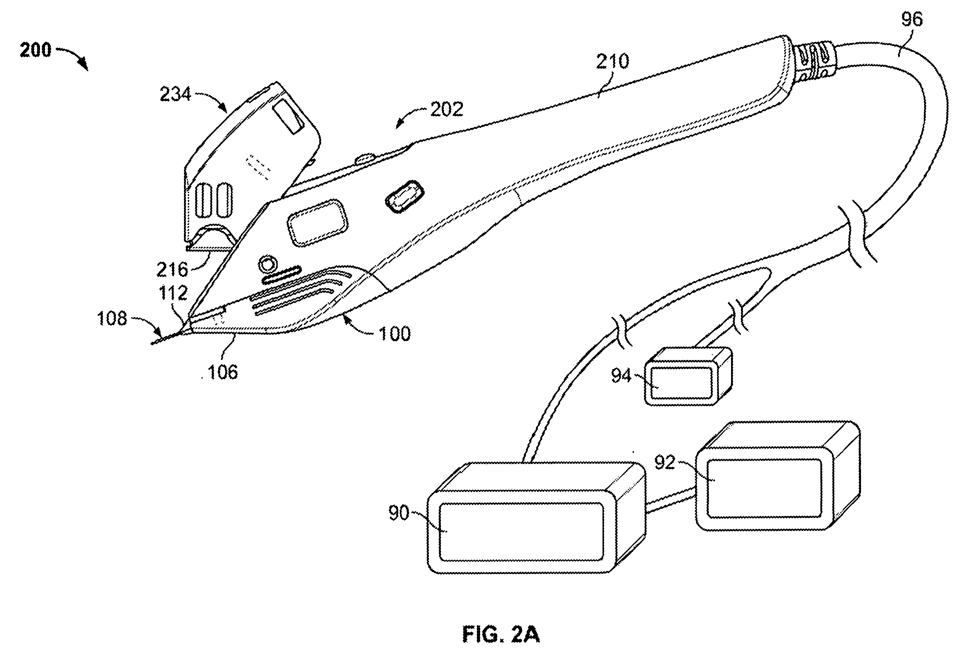

FIG. 2A illustrates a variation of a treatment system according the principles described herein. The treatment system **200** generally includes a treatment unit **202** having a hand-piece or device body **210** (or other member/feature that allows for manipulation of the system to treat tissue **10**) that is coupled to a removable cartridge **100.** The system **200** shown includes a removable cartridge **100** containing a plurality of retractable energy transfer elements arranged in an array **108.** Hereafter, the energy transfer elements are referred to as electrodes **104** and for sake of convenience can include any energy transfer element unless specifically noted otherwise. As shown, the electrodes **104** can optionally extend from a front portion **112** of the cartridge **100.** Alternatively, the electrodes **104** can extend from a front face of the device body or from any surface of the device body/cartridge.

The device body **210** is not limited to that shown. Instead, variations include device body shapes that are thinner in profile and can be held at a more vertical angle to the target tissue like a pencil or pointer. Variations also include a device body that has a loop or curved grip that facilitates one specific manner in which it can be grasped by the hand. Variations are possible especially those that ensure the physician's hand does not contact the distal end of the cartridge or the target tissue.

The devices according to the principles described herein can include any number of arrays depending upon the intended treatment site. Currently, the size of the array, as well as the number of arrays, can change depending on the variation of the invention needed. In most cases, the target region of tissue drives the array configuration. The present invention allows a physician to selectively change array configuration by attaching different cartridges **100.**

For example, a treatment unit **202** designed for relatively small treatment areas may only have a single pair of electrodes. On the other hand, a treatment unit **202** designed for use on the cheek or neck may have up to 10 electrode pairs. However, estimates on the size of the electrode array are for illustrative purposes only. In addition, the electrodes on any given array may be the same shape and profile. Alternatively, a single array may have electrodes of varying shapes, profiles, and/or sizes depending upon the intended application.

Furthermore, the array **108** defined by the individual electrodes **104** can have any number of shapes or profiles depending on the particular application. As described in additional detail herein, in those variations of the system **200** intended for skin resurfacing, the length of the electrodes **104** is generally selected so that the energy delivery occurs in the dermis layer of the skin **10** while the spacing of electrodes **104** may be selected to minimize delivery of energy between adjacent pairs of electrodes or to minimize energy to certain areas of tissue.

In those cases where the electrodes **212** are resistive, radiofrequency, microwave, inductive, acoustic, or similar type of energy transfer elements, the electrodes can be fabricated from any number of materials, e.g., from stainless steel, platinum, and other noble metals, or combinations thereof. Additionally, such electrodes may be placed on a non-conductive member (such as a polymeric member).

Additionally, the treatment unit **202** includes an actuator as described below for driving the electrode array **108** from the cartridge **100** into the target region. Examples of such actuators include, but are not limited to, gas powered cylinders, springs, linear actuators, or other such motors. Alternative variations of the system **200** include actuators driven by the control system/energy supply unit **90.**

FIG. 2A also shows an optional cooling device **234** coupled to the device body **210.** The cooling device **234** can be adjustable along the device body **210.** In the illustrated variation, the cooling device **234** is in a retracted position where it is spaced away from electrodes **108** (and thus spaced from the surface of the target tissue). This retracted position can aid the user by allowing for visualization of proper placement of the electrode array **212** into the target tissue. After the user places the device **202** on tissue, the user can advance the cooling device **234** (manually or automatically upon activation of the system) so that a cooling surface **216** of the cooling device **234** makes contact with the target tissue.

The cooling device can be an air or liquid type cooling device. Alternatively, the cooling device can include a Peltier cooling device. A Peltier cooling device can eliminate the need for a fluid source. In some cases, the cooling device can be powered using the same power supply that energizes the electrodes. Such a configuration provides a more compact design that is easier for a medical practitioner to manipulate.

The system **200** also includes an energy supply unit **90** coupled to the treatment unit **202** via a cable **96** or other means. The energy supply unit **90** may contain the software and hardware required to control energy delivery. Alternatively, the CPU, software and other hardware control systems may reside in the hand piece **210** and/or cable **96.** It is also noted that the cable **96** may be permanently affixed to the supply unit **90** and/or the treatment unit **202.** In additional variations, the hand piece **210** can contain the controls alone or the controls and the power supply necessary to delivery treatment.

In one variation, the energy supply unit **90** may be a RF energy unit. Additional energy supply units may include power supplies to provide thermal energy, ultrasound energy, laser energy, pulsed light energy, and infrared energy. Furthermore, the systems may include combinations of such energy modalities.

For example, in addition to the use of RF energy, other therapeutic methods and devices can be used in combination with RF energy to provide additional or more efficacious treatments. For example, as shown in FIG. 2A, additional energy sources **96** can be delivered via the same or additional energy transfer elements located at the working end of a treatment unit **202.** Alternatively, the radiant energy may be supplied by the energy source/supply **90** that is coupled to a diode, fiber, or other emitter at the distal end of the treatment unit **202.** The energy source/supply **94** and associated energy transfer element may comprise laser, light or other similar types of radiant energy (e.g., visible, ultraviolet, or infrared light). For example, intense pulsed light having a wavelength between 300 and 12000 nm can also be used in conjunction with RF current to heat a targeted tissue. Such associated transfer elements may comprise sources of light at the distal end of the treatment unit **202.** These transfer elements may be present on the cartridge **100,** on the device body **210** or even on the cooling unit **234**. More specifically a coherent light source or laser energy can be used in conjunction with RF to heat a targeted tissue. Examples of lasers that can be used include erbium fiber, CO₂, diode, flashlamp pumped, Nd:YAG, dye, argon, ytterbium, and Er:YAG among others. More than one laser or light source can be used in combination with RF to further enhance the effect. For example, a pulsed infra-red light source can be used to heat the skin surface, an Nd:YAG laser can be used to heat specific chromophores or dark matter below the surface of the skin, and RF current can be applied to a specific layer within or below the skin; the combination of which provides the optimal results for skin tightening, acne treatment, lipolysis, wart removal or any combination of these treatments.

Other energy modes besides or in addition to the optical energy described above can also be used in conjunction with RF current for these treatments. Ultrasound energy can be delivered either through the RF electrodes, through a face plate on the surface of the skin, or through a separate device. The ultrasound energy can be used to thermally treat the targeted tissue and/or it can be used to sense the temperature of the tissue being heated. A larger pulse of pressure can also be applied to the surface of the skin in addition to RF current to disrupt adipose tissue. Fat cells are larger and their membranes are not as strong as those of other tissue types so such a pulse can be generated to selectively destroy fat cells. In some cases, the multiple focused pressure pulses or shock waves can be directed at the target tissue to disrupt the cell membranes. Each individual pulse can have from 0.1 to 2.5 Joules of energy.

The energy supply unit **90** may also include an input/output (I/O) device that allows the physician to input control and processing variables, to enable the controller to generate appropriate command signals. The I/O device can also receive real time processing feedback information from one or more sensors associated with the device, for processing by the controller, e.g., to govern the application of energy and the delivery of processing fluid. The I/O device may also include a display, to graphically present processing information to the physician for viewing or analysis.

In some variations, the system **200** may also include an auxiliary unit **92** (where the auxiliary unit may be a vacuum source, fluid source, ultrasound generator, medication source.) Although the auxiliary unit is shown to be connected to the energy supply, variations of the system **200** may include one or more auxiliary units **92** where each unit may be coupled to the power supply **90** and/or the treatment unit **202.**

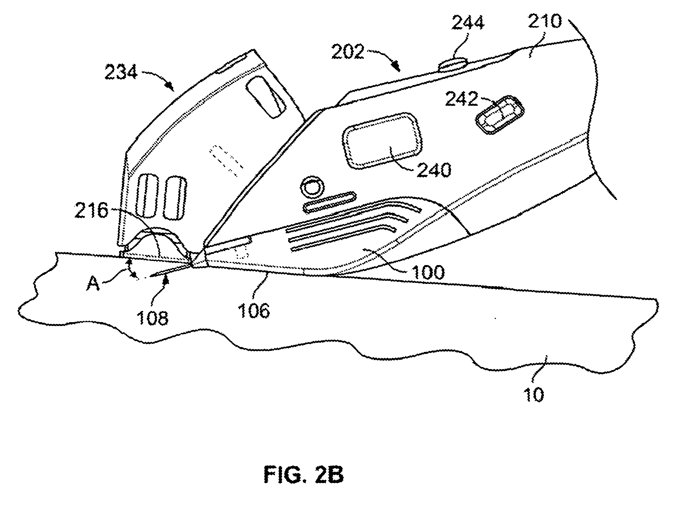

FIG. 2B illustrates a partial view of a working end of a treatment unit **202** where the treatment unit **202** engages against tissue **10** and the array **108** extends from a cartridge **100** into the tissue **10.** The cooling device **234** also engages tissue **10** so that a cooling surface **216** cools tissue directly above the area of treatment. The illustrated figure also demonstrates another feature of the system where the cartridge 100 includes a tissue engaging surface 106 having a plane that forms an angle A with a plane of the array of electrodes 108. As described below, this configuration permits a larger treatment area as well as direct cooling of the tissue surface. The devices of the present invention may have an angle A of 15 degrees. However, the angle can range from anywhere between perpendicular to parallel with respect to the tissue surface.

The tissue engaging surface 106 can also include any number of features to ensure adequate contact with tissue.

Although not shown, the tissue engagement surface may contain apertures or other features to allow improved engagement against tissue given the application of a vacuum. By drawing tissue against the tissue engaging surface the medical practitioner may better gauge the depth of the treatment. For example, given the relatively small sectional regions of the epidermis, dermis, and subcutaneous tissue, if a device is placed over an uneven contour of tissue, one electrode pair may be not be placed at the sufficient depth. Accordingly, application of energy in such a case may cause a burn on the epidermis. Therefore, drawing tissue to the tissue engaging surface of the device increases the likelihood of driving the electrodes to a uniform depth in the tissue.

In such an example, the tissue engagement surface 106 can include small projections, barbs, or even an elastic resin to increase friction against the surface of tissue. These projections or features can grip or provide friction relative to the tissue in proximity of the target tissue. This grip or friction holds the tissue in place while the electrodes are inserted at an angle relative to the grip of the projections. In another variation, the tissue engaging surface can include contact or proximity sensors to ensure that any numbers of points along the tissue engaging surface are touching the surface of the target site prior to electrode deployment and/or energy delivery.

FIG. 2B also shows the treatment unit **202** having an extension actuator **240** and a retraction actuator **242** which extend and retract the array **108** in the cartridge. The handle also contains a power control switch **244** that can start and stop delivery of energy. Clearly, the location, size, and construction of such actuators can vary. In addition, all actuators can be replaced by a single actuator. Actuation of the device can occur using a footswitch that is coupled to the control system.

As discussed below, the cooling device **234** includes a cooling plate or cooling surface **216.** Optionally, the cooling surface can have a disposable cover that prevents direct tissue contact between the actual cooling surface and the target tissue. The cover can be a disposable, sterilized component that is discarded after each treatment or after each patient.

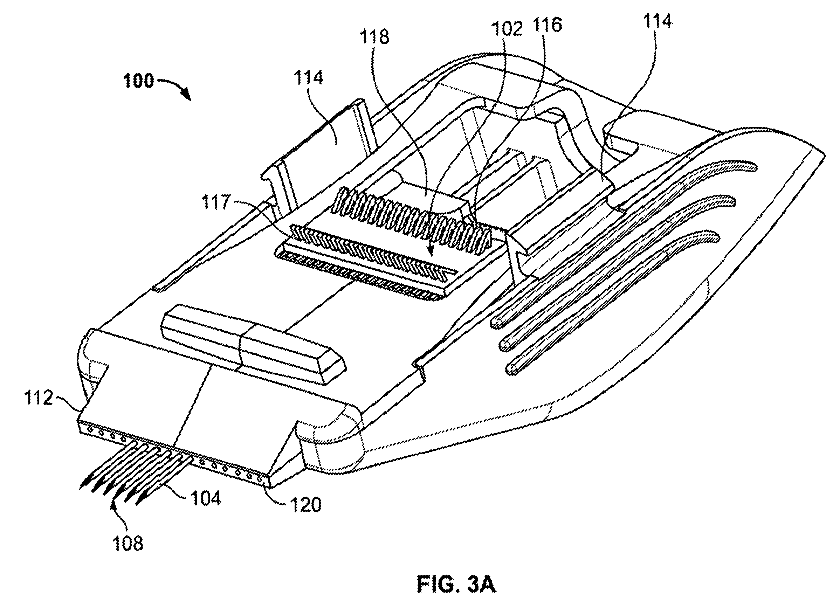

FIG. 3A illustrates one variation of a cartridge body **100** for use with the present system. As shown, the cartridge body **100** includes retention fasteners **114** allowing for coupling with the device body as well as removal from the device body. Again, any number of structures can be incorporated into the device to permit removable coupling of the cartridge body **100** to a treatment unit.

The cartridge body **100** further includes an electrode assembly **102** that is moveable or slidable within the cartridge body **100.** The mode of movement of the actuator can include sliding, rotation, incremental indexing (via a rachet-type system), stepping (via a step-motor), Accordingly, the electrode assembly **102** can include a coupling portion or structure **118** that mates with an actuating member in the device body. In the illustrated example, the electrode assembly **102** is in a treatment position (e.g., the array **108** extends from the cartridge **100** allowing for treatment). The electrode assembly **102** includes any number of electrodes **104** that form an array **108** and are extendable and retractable from a portion **104** of the cartridge **100.** As noted above, although the illustrated example shows an array **108** of 1x6 electrodes **104,** the array can comprise any dimension of M x N electrodes where the limits are driven by the nature of the treatment site as well as the type of energy delivery required.

FIG. 3A also shows the electrodes **104** in the electrode assembly **102** as having connection or contact portions **116** that couple to a connection board on a treatment unit to provide an electrical pathway from the power supply to the electrodes **104.** In the illustrated variation, the electrode assembly **102** as well as the connection portions **116** moves. This feature allows for selective connection of the electrodes with the power supply. According to the invention, the electrodes are only coupled to the power supply when in a treatment position and are incapable of delivering energy when in a retracted position. The electrode assembly and connection board may be configured to permit temperature detection at all times but only energy delivery in the treatment position. This prevents energy delivery in an unintended location, for example, when the electrodes have an insulation that only allows energy delivery at the distal tip and the intended location of energy delivery is at a specific depth in the target tissue that corresponds to the length of the extended electrode the electrode cannot delivery energy to an unintended shallower location when it is not fully extended.

The connection portions **116** can be fabricated in any number of configurations as well. For example, as shown, the connection portions **116** comprise spring contacts or spring pins of the type shown. Accordingly, the connection portions **116** can maintain contact with a corresponding contact point trace on a connection board during movement of the electrode assembly **102**

FIG. 3A also shows a front portion **112** of the cartridge **100** as having multiple guiding channels **120.** These channels **120** can support and guide the electrode **104** as they advance and retract relative to the cartridge **100.** The channels **120** can also be configured to provide alternate energy treatments to the surface of the tissue as well as suction or other fluids as may be required by a procedure. One benefit is that a single cartridge design can be configured to support a variety of electrode array configurations. For example rather than the array of six (6) electrodes as shown, the channels 120 can support any number of electrodes (the illustrated example shows a maximum of sixteen (16) but such a number is for exemplary purposes only). Furthermore, the channels **120** need not be only in a linear arrangement as shown, but could be in 1, 2, 3 or more rows or in a random configuration.

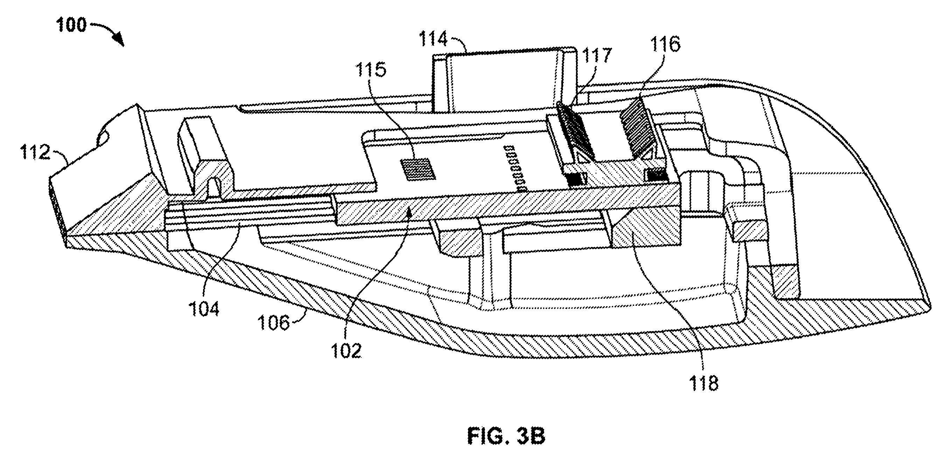

FIG. 3B illustrates a cross sectional view of a cartridge **100** when the electrode assembly **102** is in a retracted position. As shown, the connection portions **116, 117** of the electrodes **104** can extend from a top of the electrode assembly **102.** The electrode assembly 102 can also optionally include a coupling body **118** to engage an actuator on the treatment device. In this case, the electrode assembly **102** can have multiple connection portions **116, 117** per individual electrode. In such a case, the multiple connection portions **116** and **117** can be electrically insulated from one another to increase the number of configurations possible with the electrode assembly. For example, and as illustrated below, in one possible variation, the proximal connection portion **116** can electrically couple to a temperature detecting circuit on the hand unit. The distal connection portion **117** connects to a power delivery circuit only upon distal advancement of the needle assembly **102.** In such an example, the temperature of the electrodes can be continuously monitored while the power delivery to the electrodes can be limited to distal advancement of the assembly.

In another aspect of the device, Fig. 3B also shows an example of an electronic memory unit **115,** as noted above. The memory unit can provide the system with memory capabilities for containing instructions or record communication between the cartridge and hand unit and/or controller to adjust treatment parameters, monitor usage, monitor sterility, or to record and convey other system or patient characteristics. As also noted above, the unit **115** can also be an RFID antenna or receiver.

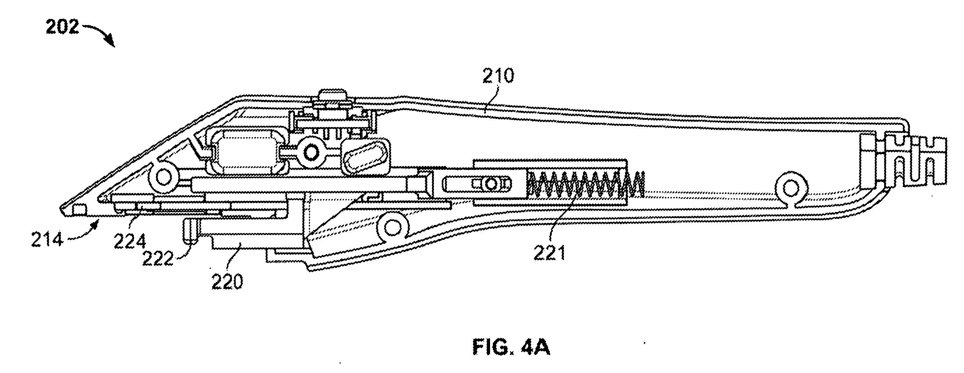

FIG. 4A illustrates a cross sectional view of a variation of a treatment unit **202** without a cartridge attached to a cartridge receiving surface **214** of the device body **210.** As shown, the device body **210** includes a moveable actuator **220** adjacent to the cartridge receiving surface **214.** In this variation, the actuator **220** is coupled to a spring **221** such that it may be spring loaded to exert sufficient force to allow penetration of the array into tissue. However, as noted above, the actuator may comprise any number of actuation means, including, but not limited to, gas powered cylinders, springs, linear actuators, or other such motors. The actuator **220** further includes an engaging surface **222** that can engage a coupler **118** (not shown) on a cartridge assembly.

The cartridge receiving surface **214** further includes a plurality of electrically conductive leads **224** that, when placed in contact with the electrode connection portions discussed above, permit energy delivery via the electrodes in the cartridge.

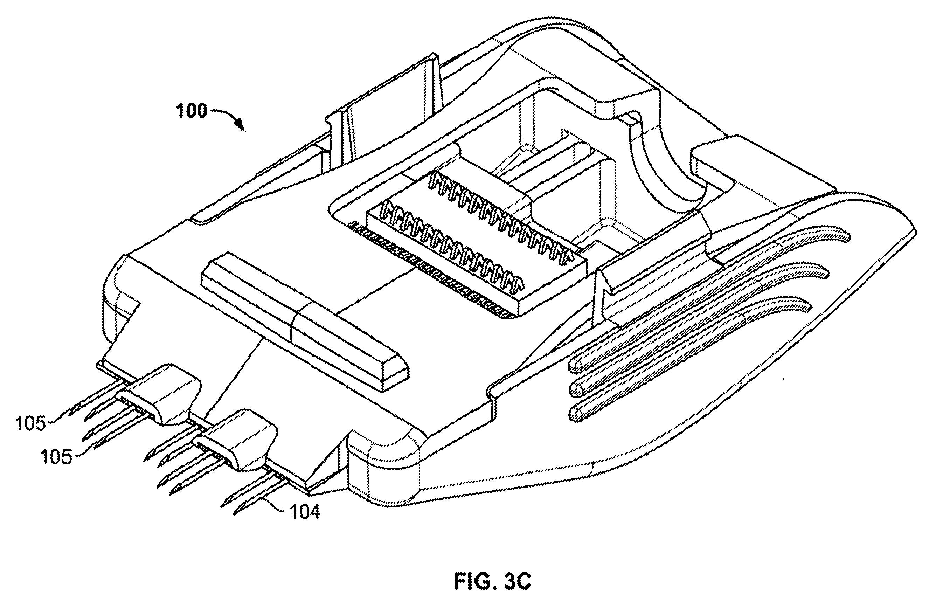

FIG. 3C shows a perspective view of another variation of an electrode assembly. In this variation, the electrodes **104** are staggered or offset such that adjacent electrode pairs **105** do not form a linear pattern. One benefit of this configuration is to overcome the creation of a 'line effect' in tissue. For example, an array of electrodes arranged in a single line can possibly result in a visible line in tissue defined by the entry points of adjacent and parallel electrodes. In the variation of Fig. 3C, staggering or offsetting the electrodes prevents the 'line effect' from occurring.

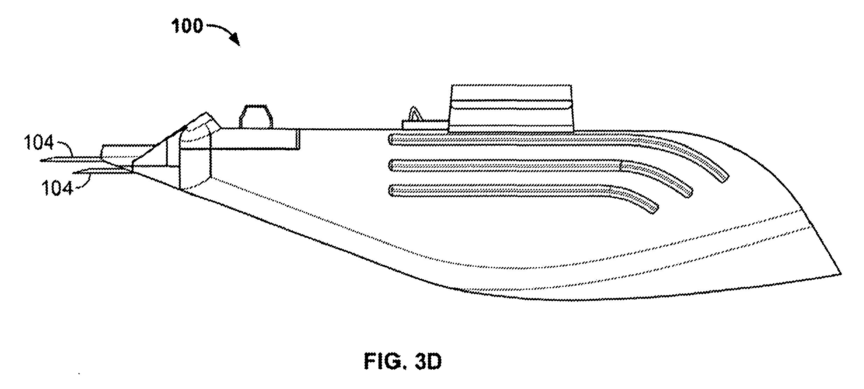

Fig. 3D shows a side view of the variation of Fig. 3C. As shown, the electrodes **104** are offset to minimize the chance of forming a single continuous line in tissue by penetration of a set of linearly arranged electrodes. Clearly, other configurations can also address the 'line effect'. For example, the spacing between adjacent electrodes can be increased to minimize a 'line effect' but to still permit efficacy of treatment. In addition, although the illustrated example shows two lines of electrodes, variations of the device include electrodes **104** that form more than two rows of electrodes.

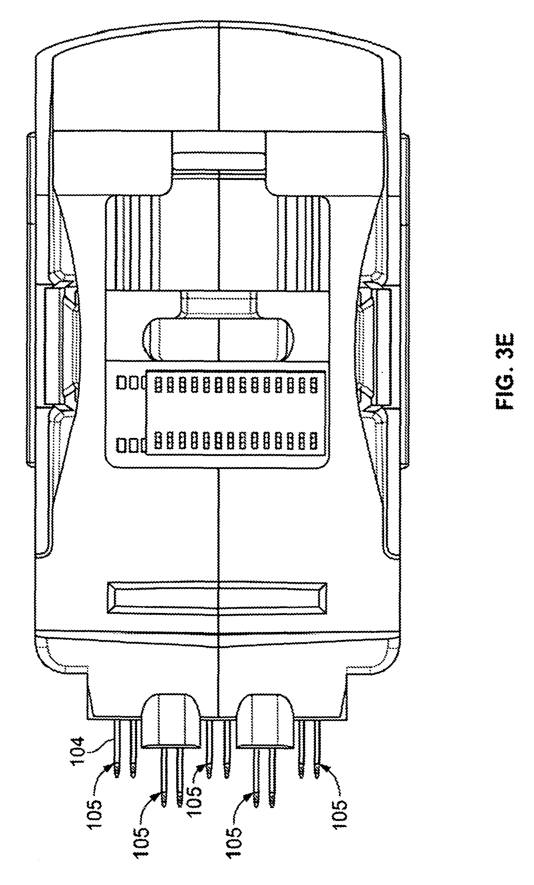

Fig 3E shows a top view of the cartridge variation of Fig. 3C. The variation illustrated shows that the plurality of electrodes comprises a plurality of electrode pairs **105.** As noted above, the electrode pairs **105** can be vertically offset from an adjacent electrode pair (as shown in Fig. 3D) so that insertion of electrode pairs into the tissue does not create a continuous line of insertion points. Moreover, and as shown in Fig. 3E the electrodes **104** can be axially offset (such that an end of the electrode) extends a greater distance than an end of an adjacent electrode or electrode pair. As noted herein, axially offsetting the electrodes allows for a uniform insertion depth when measured relative to a tissue engaging surface of the cartridge.

In one variation, each electrode pair **105** can include an active and return electrode **104** to contain current flow between electrodes in an electrode pair **105.** Alternatively, additional configurations are within the scope of the device. For instance, adjacent electrode pairs can serve as opposite poles of a circuit or the electrodes can be monopolar where the therapeutic effect is controlled by selective firing of electrodes. In additional variations, the system can be provided with a number of electrode cartridges where the spacing or offset of the electrodes varies and allows for the physician to control treatment or placement of the electrodes by exchanging cartridges.

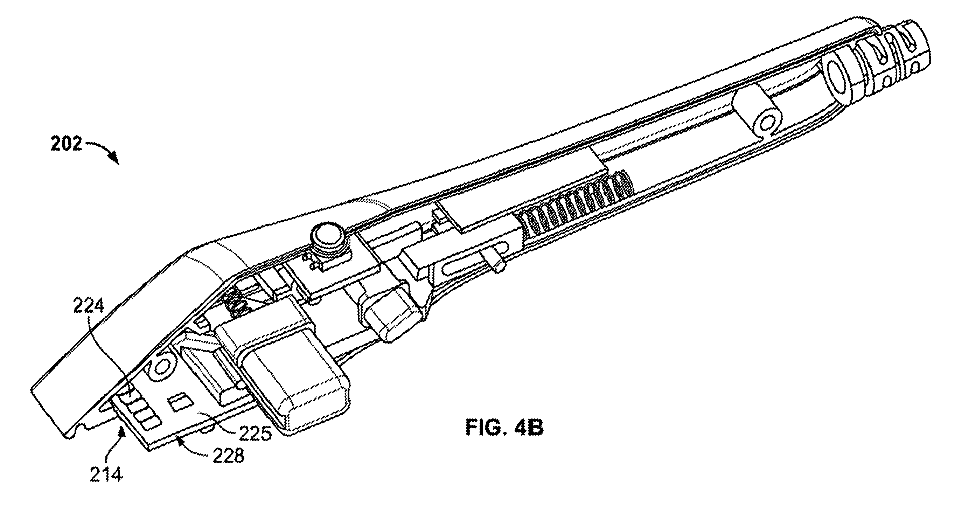

FIG. 4B shows a top perspective view of the treatment unit **202** of FIG. 4A. In this view, the back surface of the electrically conductive leads **224** are shown on a circuit or other similar type of connection board **228.** It is noted that the wires or other connection means that couple the conductive leads **224** to the power supply are omitted for purposes of illustration. The connection board can also include an electronic memory unit **225,** as noted above. Alternatively, the electronic memory unit can be included anywhere in or on the body of the hand unit. The memory unit can provide the system with memory capabilities for containing instructions or record communication between the cartridge and hand unit and/or controller to adjust treatment parameters, monitor usage, monitor sterility, or to record and convey other system or patient characteristics. As also noted above, the unit **225** can also be an RFID antenna or receiver.

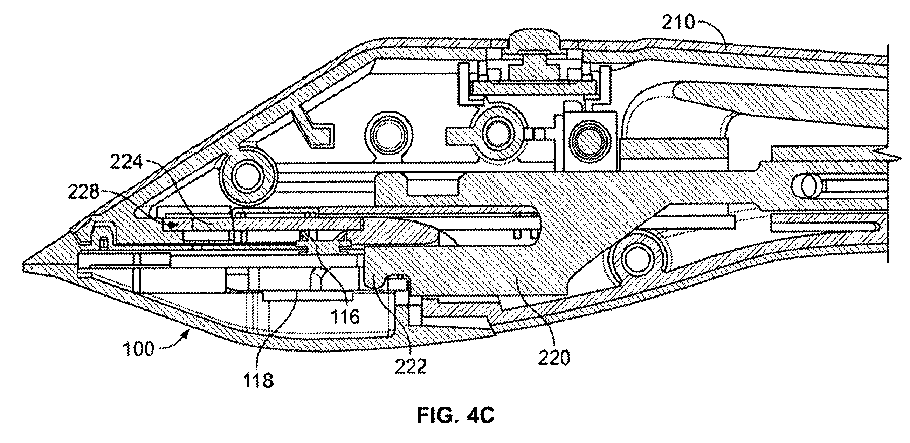

FIG. 4C shows a cross sectional view of a device body **210** coupled to a cartridge **100.** In the illustration, the electrode assembly is shown in a retracted position such that the electrode connection portions **116** do not contact the electrically conductive leads **224** on the connection board **228.** Since the engaging surface **222** and coupling portion **118** move together, distal movement of the actuator **220** causes the electrode assembly and connection portions **116** to move distally towards the treatment position. This movement causes the electrode connection portions **116** to contact the electrically conductive leads **224** allowing for energizing of the electrodes.

As noted above, using an RF energy modality, the ability to control each electrode pair on a separate channel from the power supply provides additional benefits based on the impedance or other characteristic of the tissue being treated. For example, each electrode pair may include a thermocouple to separately monitor each treatment site; the duration of the energy treatment may be controlled depending on the characteristics of the surrounding tissue; selective electrode pairs may be fired rather than all of the electrode pairs firing at once (e.g., by firing electrode pairs that are located on opposite ends of the electrode plate one can further minimize the chance that a significant amount of current flows between the separate electrode pairs.) Naturally, a number of additional configurations are also available depending on the application. Additional variations of the device may include electrode pairs that are coupled to a single channel of a power supply as well.

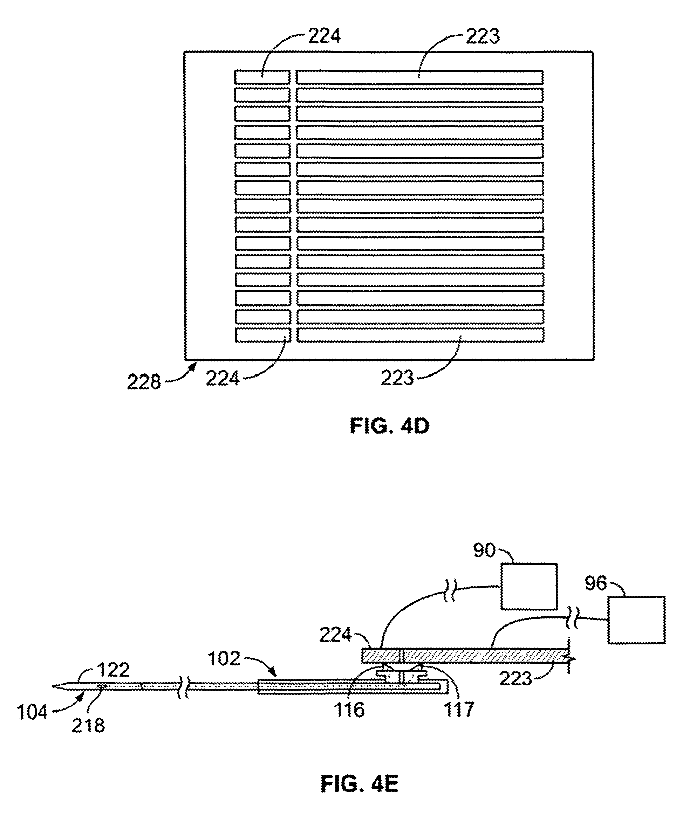

FIGS. 4D and 4E illustrates a variation of such a configuration. FIG. 4D shows a variation of the underside of a connection board **228** for use in a treatment unit **202.** In this variation, the connection board **228** includes a first set of conductive leads **224** and a second set of conductive leads **223** where the first set **224** are electrically isolated from the second set. Fig. 4E illustrates the connection board of FIG. 4D when coupled to an electrode assembly **102.** As shown, the electrode **104** in the assembly **102** includes first and second contact portions **116, 117.** When advanced into the treatment position, the first contact portion **116** engages the first conductive lead **224** while the second contact portion **117** engages the second conductive lead **223.** The first contact portion **224** can provide an electrical pathway to a power supply **90** and the second contact portion **223** can provide an electrical pathway to a temperature measuring unit whether a stand alone unit or a temperature measuring unit coupled to the controller **90.** Accordingly, such a configuration permits monitoring of temperature (e.g., via a temperature detecting element **218**) continuously but limiting powering of the electrode **104** only when the electrode assembly is in a treatment position (as described herein).

The present device may deliver energy based upon sensing tissue temperature conditions as a form of active process feedback control. Alternatively, the device may monitor changes in impedance of the tissue being treated and ultimately stop the treatment when a desired value is obtained. In another variation, the delivery of energy can depend on whether impedance is within a certain range. Such impedance monitoring can occur during energy delivery and attenuate power if the dynamically measured impedance starts to exceed a given value or if the rate of increase is undesirably high. Yet another mode of energy delivery is to provide a total maximum energy over a duration of time.

As noted herein, temperature or other sensing may be measured beneath the epidermis in the dermis region. Each probe or electrode may include a sensor or the sensor may be placed on a structure that penetrates the tissue but does not function as an energy delivery electrode. In yet another variation, the sensors may be a vertically stacked array (i.e. along the length of the electrode) of sensors to provide data along a depth or length of tissue.

Energizing the RF electrodes in the dermal layer produces a healing response caused by thermally denaturing the collagen in the dermal layer of a target area. As noted herein, systems according to the present invention are able to provide a desirable effect in the target area though they use a relatively low amount of energy when compared to systems that treat through the epidermis. Accordingly, systems of the present invention can apply energy in various modes to improve the desired effect at the target area.

In one mode, the system can simply monitor the amount of energy being applied to the target site. This process involves applying energy and maintaining that energy at a certain pre-determined level. This treatment can be based on a total amount of energy applied and/or application of a specific amount of energy over a set period of time. In addition, the system can measure a temperature of the target site during the treatment cycle and hold that temperature for a pre-determined amount of time. However, in each of these situations, the system does not separate the time or amount of energy required to place the target site in the desired state from the time or amount of energy required to hold the target site in the desired state. As a result, the time or amount of energy used to place the target in a desired state (e.g., at a pre-determined temperature) is included in the total treatment cycle. In some applications, it may be desirable to separate the portion of the treatment cycle required to elevate the target to a pre-determined condition from the portion of the treatment cycle that maintains the target site at the pre-determined conditions.

For example, in one variation, the system can maintain a temperature of the target site at a pre-determined treatment temperature during a pre-determined cycle or dwell time. The system then delivers energy to maintain the target site at the treatment temperature. Once the target site reaches the treatment temperature, the system then maintains this condition for the cycle or dwell time. This variation allows for precise control in maintaining the target site at the pre-determined temperature. In another variation, the system can monitor the amount of power applied to the target site for a specific dwell time. By continuously measuring current and output voltage, the system can calculate both the impedance changes and the delivered power levels. With this method a specific amount of power can be delivered to the target tissue for a specified amount of time. In addition, the above variations can be combined with various methods to control time, temperature or energy parameters to place the tissue in the desired state. For example, the system can employ a specified ramp time or maximum energy to achieve the pre-determined treatment temperature. Such a variation can create a faster or slower ramp to the treatment temperature.

Although the treatment of tissue generally relies on energy to affect the tissue, the mere act of inserting the electrode array into tissue can also yield therapeutic benefits. For instance, the mechanical damage caused by placement of the electrodes also produces an adjunct healing response. The healing response to injury in the skin tissue can contribute to the production of new collagen (collagenesis) that can further improve the tone or appearance of the skin. Accordingly, in one variation a medical practitioner may opt to use the systems to create mechanical injury to tissue by placing electrodes into target areas without thermal treatment to induce a healing response in the targeted area.

The low energy requirements of the system present an additional advantage since the components on the system undergo less stress than those systems needing higher amounts of energy. In those systems requiring higher energy, RF energy is often delivered in a pulsed fashion or for a specific duty cycle to prevent stressing the components of that system. In contrast, the reduced energy requirements of the present system allow for continual delivery of RF energy during a treatment cycle. In another variation, the duty cycle of variations of the present system can be pulsed so that temperature measurements can be taken between the pulsed deliveries of energy. Pulsing the energy delivery allows for an improved temperature measurement in the period between energy deliveries and provides precise control of energy delivery when the goal of the energy delivery is to reach a pre-determined temperature for a pre-determined time.

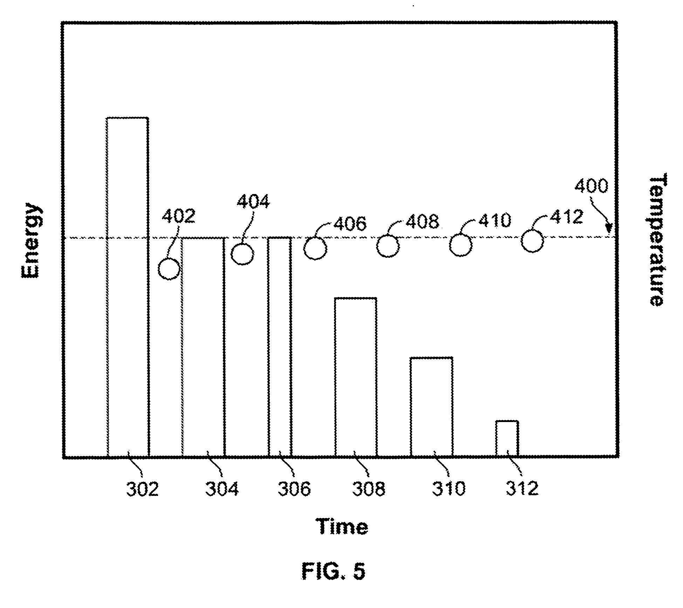

FIG. 5 illustrates a graph of energy delivery and temperature versus time. As shown, the pulses or cycles of energy are represented by the bars **302, 304, 306, 308, 310, 312.** Each pulse has a parameter, including amount of energy, duration, maximum energy delivered, energy wave form or profile (square wave, sinusoidal, triangular), current, voltage, amplitude, frequency. As shown in the graph, measurements are taken between pulses of energy. Accordingly, between each pulse of energy delivery one or more temperature sensor(s) near the electrode obtains a temperature measurement **402, 404, 406, 408, 410, 412.** The controller compares the measured temperature to a desired temperature (illustrated by **400**). Based on the difference, the energy parameters are adjusted for the subsequent energy pulse. Measuring temperature between pulses of energy allows for a temperature measurement that is generally more accurate than measuring during the energy delivery pulse. Moreover, measuring between pulses allows for minimizing the amount of energy applied to obtain the desired temperature at the target region.

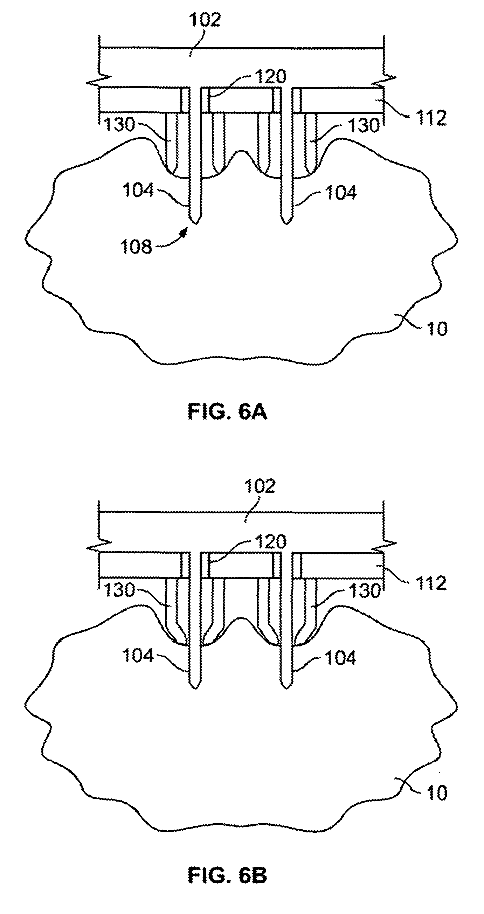

FIG. 6A illustrates an aspect for use with the variations of the devices described herein. In this example, the electrodes **104** advance through an introducer member or cannula **130** located on the front face **112** of a cartridge. The cannula **130** places tissue **10** in a state of tension (also called 'traction'). In this variation the introducer/cannula **130** is located about each channel **120** in the cartridge.

As shown, once the introducer member **130** engages tissue **10,** the tissue first elastically deforms as shown. Eventually, the tissue can no longer deflect and is placed in traction by the introducer members **130.** As a result, the electrodes **104** more readily penetrate the tissue.

FIG. 6B illustrates another variation of the introducer member **130** that is tapered inwards toward the electrodes so that the opening at the distal end closely fits around the electrode.

In another variation, insertion of the array **108** can consist of 2 or more steps. In the first step the actuation of the extension presses the channels **120** against the target tissue to create a state of traction. Further actuation advances the array **108** through the channels **120** and into the target tissue. Since the target tissue is under traction, the array requires less force to penetrate the tissue. In another variation, the channels **120** can be individual cannulae that extend from the distal face of the cartridge. Such a configuration produces traction on a smaller portion of target tissue. Alternatively, the two step extension process can be composed of a first step which extends small projections out of the tissue engaging surface of the cartridge in a direction that is substantially opposite of the direction of electrode extension which occurs in the second step. This alternative creates more traction which further eases insertion of the electrodes as the target tissue is stretched in opposite directions.

In a device using an RF energy modality, the electrodes **104** can be arranged in a pair configuration. In a bi-polar configuration one electrode serves a first pole, while the second electrode serves as the second pole (it is also common to refer to such electrodes as the active and return electrodes). The spacing of electrode pairs is sufficient so that the pair of electrodes is able to establish a treatment current path therebetween for the treatment of tissue. However, adjacent electrode pairs can be spaced sufficiently to minimize the tendency of current flowing between the adjacent pairs. Typically, each electrode pair is coupled to a separate power supply or to a single power supply having multiple channels for each electrode pair.

The benefit of such a configuration is that, when compared to conventional treatments, the amount of power required to induce heating in the target tissue is much reduced. For example, because the electrodes are spaced to provide heating across the electrode pairs at the target tissue, each channel of the system may provide as little as 1 watt of energy to produce the desired temperature increase at the site. In additional variations, the amount of energy may be no more than 3 or 5 watts. However, any amount of energy necessary to accomplish the desired effect is possible. In contrast, if a treatment system delivered energy over the entire electrode array, a much greater amount of energy would be required to generate the desired temperature over the larger surface area of tissue. Moreover, the energy demand is less because the treatment applies energy directly to the target tissue rather than though additional layers of tissue.

In one variation of the device, it is believed that a desirable spacing of the first and second electrode poles is between I and 3 mm, while a desirable spacing of electrode pairs is between 5 and 6 mm. In one example, the described configuration allowed for each independent channel to deliver no more than 1 watt, 3 watts, 5, watts or any other amount of energy to deliver acceptable tissue treatment results. Obviously, the power supply may be configured to deliver greater amounts of energy as needed depending on the application.

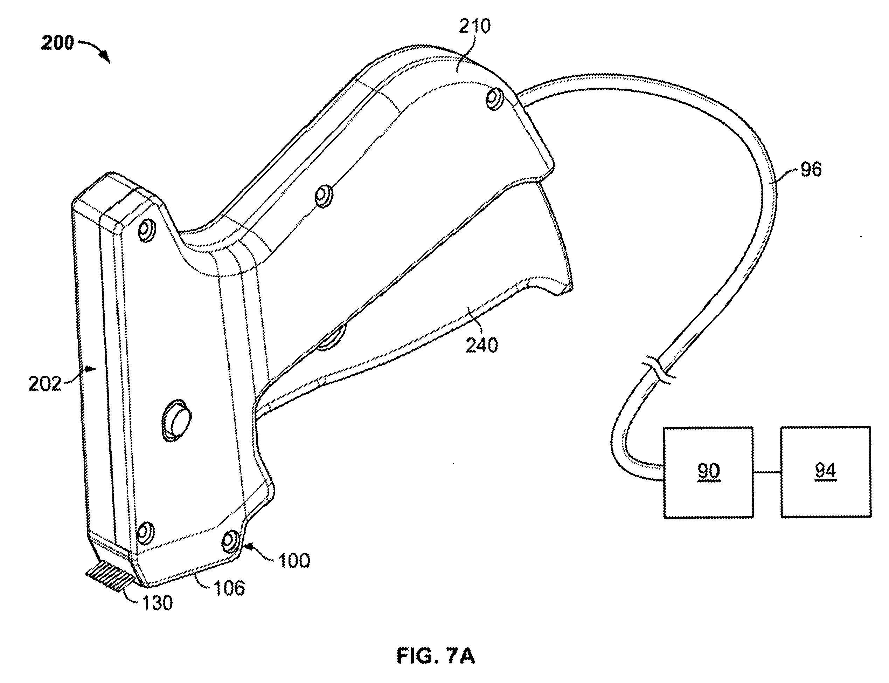

FIG. 7A illustrates another variation of a system **200** for use in accordance with the principles discussed herein. In this variation, the system **200** includes a treatment unit **202** having a cartridge **100** from which a cannula or introducer member **130** extends at an oblique angle relative to a tissue engagement surface **106.** As described below, the ability to insert the electrodes (not shown) into the tissue at an oblique angle increases the treatment area and allows for improved cooling at the tissue surface. Although the variation only shows a single array of introducers for electrodes, variations of the invention may include multiple arrays of electrodes. In addition, the devices and systems described below may be combined with the features described herein to allow for improved penetration of tissue. The devices of the present invention may have an angle **A** of 15 degrees. However, the angle may be anywhere ranging between 5 and 85 degrees.

Although the introducer member **130** is shown as being stationary, variations of the device include introducer members that are slidable on the electrodes. For example, to ease insertion of the electrode, the electrode may be advanced into the tissue. After the electrode is in the tissue, the introducer member slides over the electrode to a desired location. Typically, the introducer member is insulated and effectively determines the active region of the electrode. In a device using RF energy, the introducer member may have a return electrode on its tip. Accordingly, after it advances into the tissue, application of energy creates a current path between the electrode and the return electrode on the introducer.

The treatment unit **202** of the device **200** also includes a handle portion **210** that allows the user to manipulate the device **200.** The handle portion **210** includes a lever or lever means **240** that actuates the electrodes into the tissue (as discussed in further detail below).

As discussed above, the device **200** can be coupled to a power supply **90** with or without an auxiliary unit **94** via a connector or coupling member **96.** In some variations of the device, a display or user interface can be located on the body of the device **200** as discussed below.

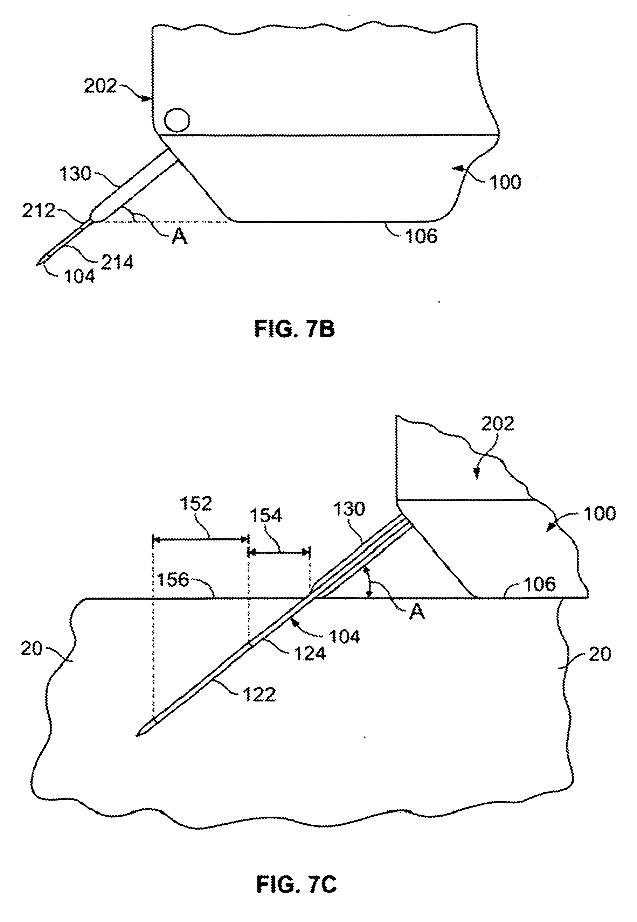

FIG. 7B illustrates a partial side view of the electrodes **104** and tissue engaging surface **106** of the electrode device of FIG. 7A. As shown, the electrodes **104** extend from the cartridge **100** through the introducer **130.** In alternate variations, the electrodes can extend directly from the body of the device or through extensions on the device.

As shown, the electrodes **104** are advanceable from the cartridge (in this case through the introducers **130**) at an oblique angle **A** as measured relative to the tissue engagement surface **106.** The tissue engagement surface **106** allows a user to place the device on the surface of tissue and advance the electrodes **104** to the desired depth of tissue. Because the tissue engagement surface **106** provides a consistent starting point for the electrodes, as the electrodes **104** advance from the device **202** they are driven to a uniform depth in the tissue.

For instance, without a tissue engagement surface, the electrode **104** may be advanced too far or may not be advanced far enough such that they would partially extend out of the skin. As discussed above, either case presents undesirable outcomes when attempting to treat the dermis layer for cosmetic effects. In cases where the device is used for tumor ablation, inaccurate placement may result in insufficient treatment of the target area.

FIG. 7C illustrates a magnified view of the electrode entering tissue **20** at an oblique angle **A** with the tissue engaging surface **106** resting on the surface of the tissue **20.** As is shown, the electrode **104** can include an active area **122.** Generally, the term 'active area' refers to the part of the electrode through which energy is transferred to or from the tissue. For example, the active area could be a conductive portion of an electrode, it can be a resistively heated portion of the electrode, or even comprise a window through which energy transmits to the tissue. Although this variation shows the active area **122** as extending over a portion of the electrode, variations of the device include electrodes **104** having larger or smaller active areas **122.**

In any case, because the electrodes **104** enter the tissue at an angle **A,** the resulting region of treatment **152,** corresponding to the active area **122** of the electrode is larger than if the needle were driven perpendicular to the tissue surface. This configuration permits a larger treatment area with fewer electrodes **104.** In addition, the margin for error of locating the active region **122** in the desired tissue region is greater since the length of the desired tissue region is greater at angle **A** than if the electrode were deployed perpendicularly to the tissue.

As noted herein, the electrodes **104** may be inserted into the tissue in either a single motion where penetration of the tissue and advancement into the tissue are part of the same movement or act. However, variations include the use of a spring mechanism or impact mechanism to drive the electrodes **104** into the tissue. Driving the electrodes **104** with such a spring-force increases the momentum of the electrodes as they approach tissue and facilitates improved penetration into the tissue. As shown below, variations of the devices discussed herein may be fabricated to provide for a dual action to insert the electrodes. For example, the first action may comprise use of a spring or impact mechanism to initially drive the electrodes to simply penetrate the tissue. Use of the spring force or impact mechanism to drive the electrodes may overcome the initial resistance in puncturing the tissue. The next action would then be an advancement of the electrodes so that they reach their intended target site. The impact mechanism may be spring driven, fluid driven or driven via other means known by those skilled in the art. One possible configuration is to use an impact or spring mechanism to fully drive the electrodes to their intended depth.

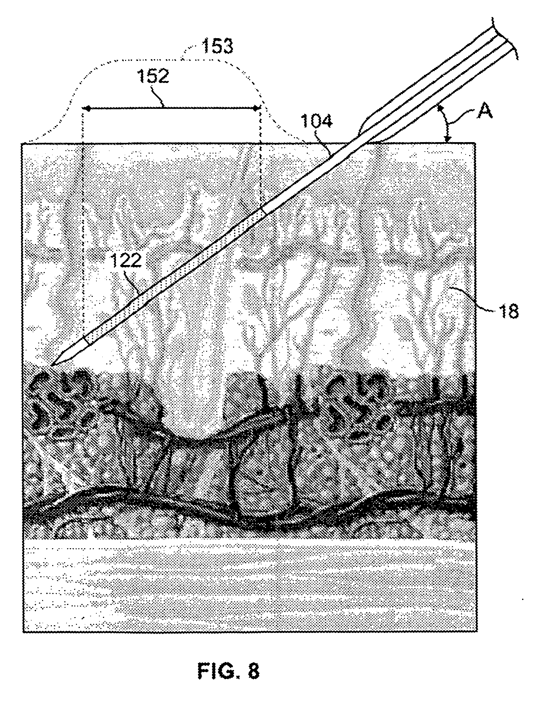

FIG. 8 illustrates an example of the benefit of oblique entry when the device is used to treat the dermis **18.** As shown, the length of the dermis **18** along the active region **122** is greater than a depth of the dermis **18.** Accordingly, when trying to insert the electrode in a perpendicular manner, the shorter depth provides less of a margin for error when trying to selectively treat the dermis region **18.** As discussed herein, although the figure illustrates treatment of the dermis to tighten skin or reduce winkles, the device may be used to affect skin anomalies **153** such as acne, warts, sebaceous glands, tattoos, or other structures or blemishes. In addition, the electrode may be inserted to apply energy to a tumor, a hair follicle, a fat layer, adipose tissue, SMAS, a nerve or a pain fiber or a blood vessel.

Inserting the electrode at angle **A** also allows for direct cooling of the surface tissue. As shown in FIG. 7C, the area of tissue on the surface **156** that is directly adjacent to or above the treated region **152** (i.e., the region treated by the active area **122** of the electrode **104**) is spaced from the entry point by a distance or gap **154.** This gap **154** allows for direct cooling of the entire surface **156** adjacent to the treated region **152** without interference by the electrode or the electrode mounting structure. In contrast, if the electrode were driven perpendicularly to the tissue surface, then cooling must occur at or around the perpendicular entry point.

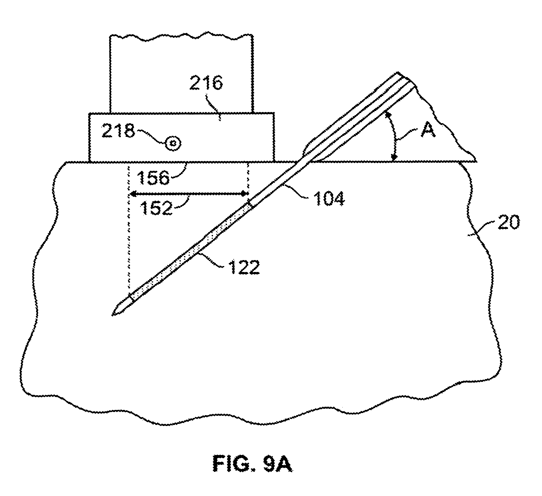

FIG. 9A illustrates one example of a cooling surface **216** placed on body structure or tissue **20.** As shown, the electrode **104** enters at an oblique angle **A** such that the active region **122** of the electrode **104** is directly adjacent to or below the cooling surface **216.** In certain variations, the cooling surface **216** may extend to the entry point (or beyond) of the electrode **104.** However, it is desirable to have the cooling surface **216** over the electrode's active region **122** because the heat generated by the active region **122** will have its greatest effect on the surface at the surface location **156.** In some variations, devices described herein may also incorporate a cooling source in the tissue engagement surface.

The cooling surface **216** and cooling device may be any cooling mechanism known by those skilled in the art. For example, it may be a manifold type block having liquid or gas flowing through for convective cooling. Alternatively, the cooling surface **216** may be cooled by a thermoelectric cooling device (such as a fan or a Peltier-type cooling device). In such a case, the cooling may be driven by energy from the electrode device thus eliminating the need for additional fluid supplies. One variation of a device includes a cooling surface **216** having a temperature detector **218** (thermocouple, RTD, optical measurement, or other such temperature measurement device) placed within the cooling surface. The device may have one or more temperature detectors **218** placed anywhere throughout the cooling surface **216** or even at the surface that contacts the tissue.

In one application, the cooling surface **216** is maintained at or near body temperature. Accordingly, as the energy transfer occurs causing the temperature of the surface **156** to increase, contact between the cooling surface **216** and the tissue **20** shall cause the cooling surface to increase in temperature as the interface reaches a temperature equilibrium. Accordingly, as the device's control system senses an increase in temperature of the cooling surface **216**, additional cooling can be applied thereto via increased fluid flow or increased energy supplied to a Peltier-type device. The cooling surface can also pre-cool the skin and underlying epidermis prior to delivering the therapeutic treatment. Alternatively, or in combination, the cooling surface can cool the surface and underlying epidermis during and/or subsequent to the energy delivery where such cooling is intended to maintain the epidermis at a specific temperature below that of the treatment temperature. For example the epidermis can be kept at 30 degrees C when the target tissue is raised to 65 degrees C.

When treating the skin, it is believed that the dermis should be heated to a predetermined temperature condition, at or about 65 degree C, without increasing the temperature of the epidermis beyond 42 degree C. Since the active area of the electrode is designed to remain beneath the epidermis, the present system applies energy to the dermis in a targeted, selective fashion, to dissociate and contract collagen tissue. By attempting to limit energy delivery to the dermis, the configuration of the present system also minimizes damage to the epidermis.

The cooling surface may comprise any commonly known thermally conductive material, metal, or compound (e.g., copper, steel, aluminum). Variations of the devices described herein may incorporate a translucent or even transparent cooling surface. In such cases, the cooling device will be situated so that it does not obscure a view of the surface tissue above the region of treatment.

In one variation, the cooling surface can include a single crystal aluminum oxide (Al₂O₃). The benefit of the single crystal aluminum oxide is a high thermal conductivity, optical clarity, ability to withstand a large temperature range, and the ability to fabricate the single crystal aluminum oxide into various shapes. A number of other optically transparent or translucent substances could be used as well (e.g., diamond, other crystals or glass).

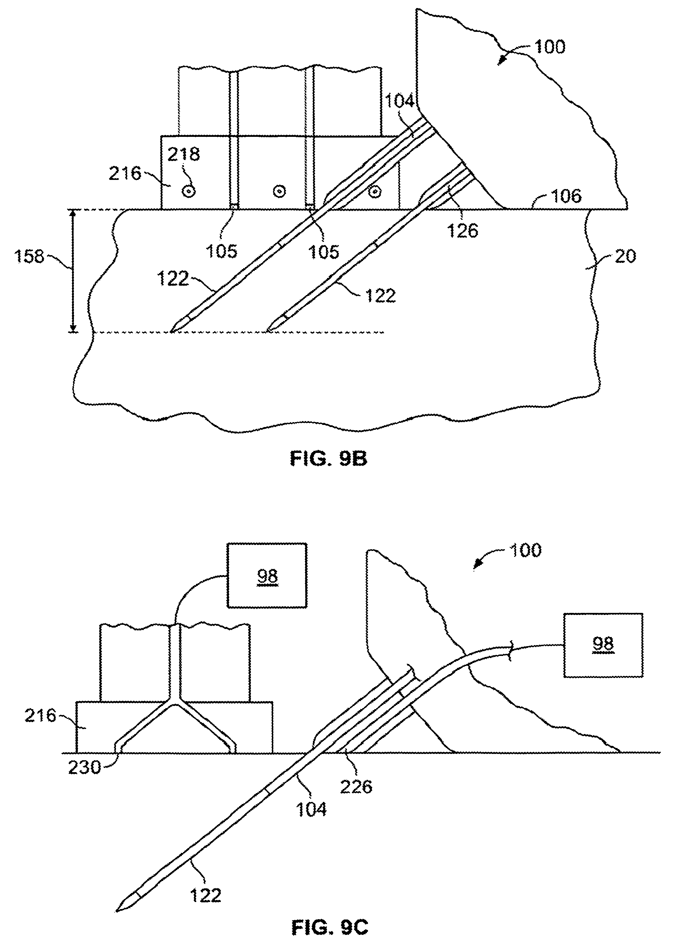

FIG. 9B illustrates another aspect for use with variations of the devices described herein. In this variation, the cartridge **100** includes two arrays of electrodes **104, 126.** As shown, the first plurality **104** is spaced evenly apart from and parallel to the second plurality **126** of electrodes. In addition, as shown, the first set of electrodes **104** has a first length while the second set of electrodes **126** has a second length, where the length of each electrode is chosen such that the sets of electrodes **104, 126** extend into the tissue **20** by the same vertical distance or length **158.** Although only two arrays of electrodes are shown, variations of the invention include any number of arrays as required by the particular application. In some variations, the lengths of the electrodes **104, 126** are the same. However, the electrodes will be inserted or advanced by different amounts so that their active regions penetrate a uniform amount into the tissue. As shown, the cooling surface may include more than one temperature detecting element **218.**

FIG. 9B also illustrates a cooling surface **216** located above the active regions **122** of the electrodes. In such a variation, it may be necessary for one or more of the electrode arrays to pass through a portion of the cooling surface **216.** Alternative variations of the device include electrodes that pass through a portion of the cooling device (such as the Peltier device described below).

FIG. 9B also shows a variation of the device having additional energy transfer elements 105 located in the cooling surface **216.** As noted above, these energy transfer elements can include sources of radiant energy that can be applied either prior to the cooling surface contacting the skin, during energy treatment or cooling, or after energy treatment

FIG. 9C shows an aspect for use with devices of the invention that allows marking of the treatment site. As shown, the cartridge 100 may include one or more marking lumens **226, 230** that are coupled to a marking ink **98.** During use, a medical practitioner may be unable to see areas once treated. The use of marking allows the practitioner to place a mark at the treatment location to avoid excessive treatments. As shown, a marking lumen **226** may be placed proximate to the electrode **104.** Alternatively, or in combination, marking may occur at or near the cooling surface **216** since the cooling surface is directly above the treated region of tissue. The marking lumens may be combined with or replaced by marking pads. Furthermore, any type of medically approved dye may be used to mark. Alternatively, the dye may comprise a substance that is visible under certain wavelengths of light. Naturally, such a feature permits marking and visualization by the practitioner given illumination by the proper light source but prevents the patient from seeing the dye subsequent to the treatment.

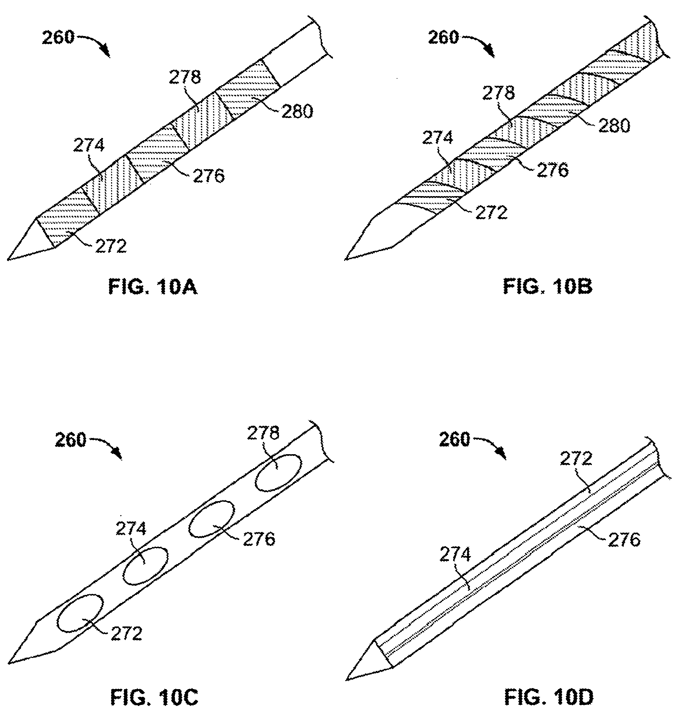

FIGS. 10A-10D illustrate variations of electrodes for use with the systems described herein. Depending upon the application, it may be desirable to provide an electrode 260 that has a variable resistance along the active region of the electrode **260.** FIGS. 10A-10D illustrate a partial example of such electrodes. As shown in FIG. 10A and 10B, an electrode may have concentric or spiral bands that create varying ranges of impedance **272, 274, 276, 278,** and **280** along the electrode **260.** In addition, as shown in FIG. 10C, the electrode **260** may have regions **272, 274,** and **276** and 278 along the electrode of varying resistance. FIG. 10D illustrates a similar concept where the regions of resistance **272, 274,** and **276,** run in longitudinal stripes along the electrode 260. These configurations may be fabricated through spraying, dipping, plating, anodizing, plasma treating, electro-discharge, chemical applications, etching.

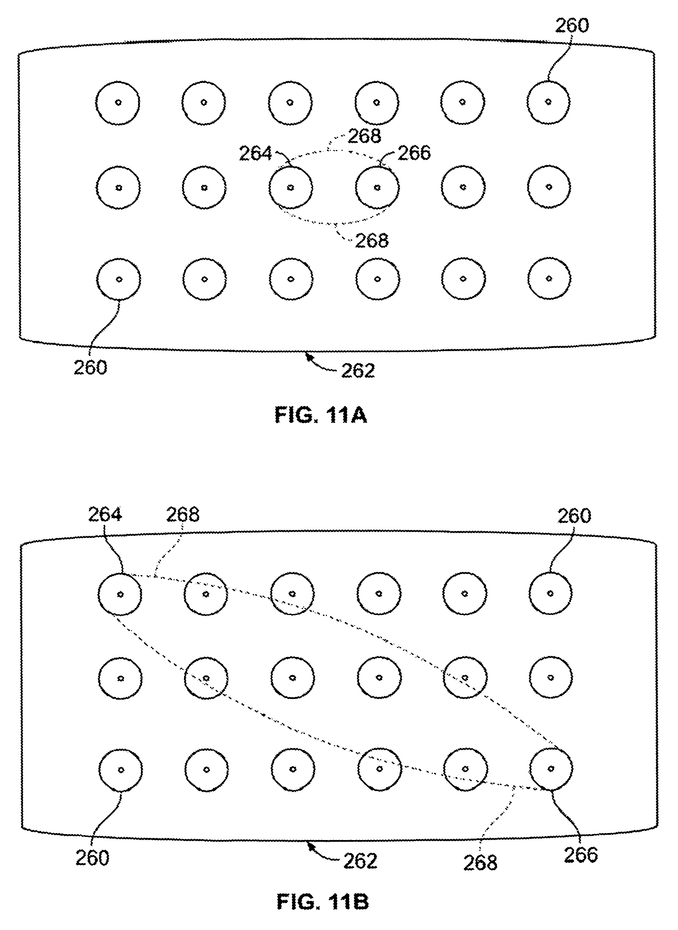

FIGS. 11A-11B illustrate examples of system configurations that can be incorporated into any conventional electrode array or into the devices described above using RF energy. As shown, in this example the electrode array **262** comprises a 3x6 array of electrodes. However, the selective pairing of electrodes can occur with a 1 x N array as well as an M x N array. Each electrode in the array **262** is configured to energize separately. This configuration provides the ability of any given pair of electrodes to form a circuit for treating tissue. In one example, in the variation of FIG. 11A, the power supply energizes adjacent electrode pairs **264, 266.** This configuration generates the smallest treatment area in the electrode array **262.** FIG. 11B illustrates a situation where the farthest electrode pairs **264, 266** within the array **262** are triggered to form a current path **268.** One benefit of this configuration is that a single electrode array may form a number of patterns based on various combinations of pairs that may be formed in the array. The array may be able to provide a denser treatment or more uniform tissue heating. The treatment can deliver targeted therapy to key areas of tissue. In one variation, various pairs of the electrode array may be triggered sequentially during a single insertion.

Although the systems described herein may be used by themselves, the devices described above can be used in combination with substances such as moisturizers, ointments that increase the resistivity of the epidermis. Accordingly, prior to the treatment, the medical practitioner can prepare the patient by increasing the resistivity of the epidermis. During the treatment, because of the increased resistivity of the epidermis, energy would tend to flow in the dermis.

In addition, such substances can be combined with various other energy delivery modalities to provide enhanced collagen production in the targeted tissue or other effects as described herein.

In one example, 5-aminolevulinic acid (ALA) or other photolabile compounds can be used that generate a biologically active agent when present in the skin upon exposure to sunlight or other applied spectrums of activating light. Coatings or ointments can also be applied to the skin surface in order to stabilize the soft tissue. Temporarily firming or stabilizing the skin surface will reduce skin compliance and facilitate the insertions of the electrodes of the current device. An agent such as cyanoacrylate, spirit gum, latex, a facial mask or other substance that cures into a rigid or semi-rigid layer can be used to temporarily stabilize the skin. The topical ointments or coatings can be applied to enhance collagen production or to stabilize the skin for ease of electrode insertion or both. Furthermore, topical agents can be applied to alter the electrical properties of the skin. Applying an agent which increases the impedance of the epidermal layer will reduce the conductance of RF current through that layer and enhance the conductance in the preferred dermal layer. A topical agent that penetrates the epidermal layer and is absorbed by the dermal layer can be applied that lowers the impedance of the dermal layer, again to enhance the conduction of RF current in the dermal layer. A topical agent that combines both of these properties to affect both the dermal and epidermal layers conductance can also be used in combination with RF energy delivery.

In addition to topical agents, the device with its penetrating electrodes lends itself to the delivery of agents and materials directly to a specific region of tissue. For example, anesthetic agents such as lidocaine can be delivered through the electrode cannula to the dermis and epidermis to deaden nerve endings prior to the delivery of therapeutic energy. Collagen or other filler material can be delivered prior to, during or after energy delivery. Botulinum Toxin Type A, Botox, or a similar neurotoxin can be delivered below the skin layer to create temporary paralysis of the facial muscles after energy delivery. This may be provide a significant improvement in the treatment results as the muscles would not create creases or wrinkles in the skin while the thermally treated collagen structure is remodeled and collagenesis occurs.

Another means to enhance the tissue's therapeutic response is the use of mechanical energy through massage. Such an application of mechanical energy can be combined with the systems described herein. Previously, devices have used massaging techniques to treat adipose tissue. For example U.S., Patent No 5,961,475 discloses a massaging device that applies negative pressure as well as massage to the skin. Massage both increases blood circulation to the tissue and breaks done connections between the adipose and surrounding tissue. For example, these effects may be combined with energy treatment of the tissue to enhance the removal of fat cells.

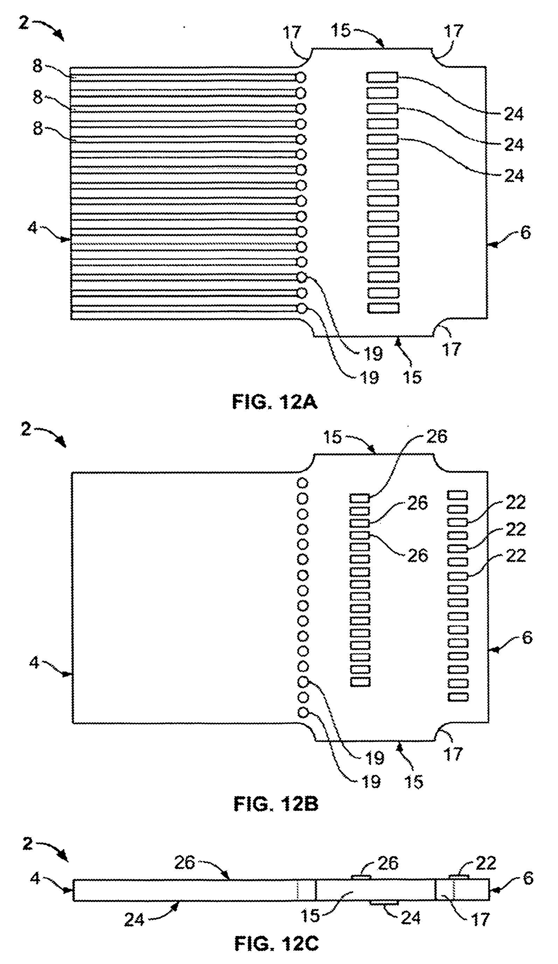

Referring to Figure 12A, a printed circuit board **2** is shown which is comprised of a distal end **4,** a proximal end **6,** and two side projections **15** that have engagement features **17.** The engagement features **17** and side projections **15** allow the connection of the PCB **2** to a carrier or delivery device (not shown). The printed circuit board **2** or PCB contains multiple slots **8** that are lined with conductive lead material that extends to the distal end **4** of the PCB **2.** The PCB **2** also includes multiple corresponding through holes 19 which allow the multiple conductive leads in the slots **8** to be electrically connected to electrical traces (not shown) on other layers of the PCB **2.** Some of those traces are also in electrical connection to the multiple connector leads **24.**

Referring now to Figure 12B, the top side of the PCB 2 shown in Figure 12A reveals additional multiple connector leads **26** and **22.** These leads can also or alternately be in electrical connection to the leads in the multiple through holes **19.** Figure 12C shows the PCB 2 from the side. It can be seen from this view that all of the multiple connector leads **24, 26, 22** are slightly raised from the main body of the PCB **2**

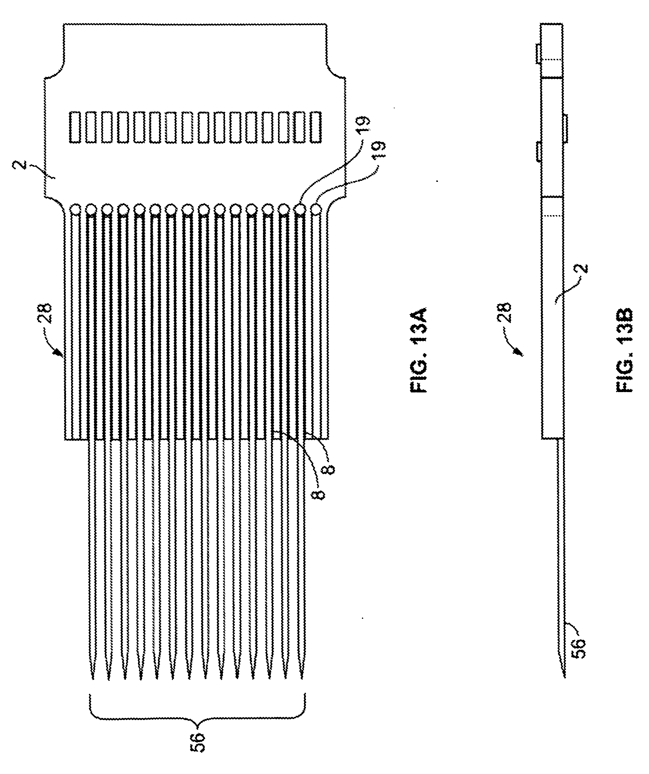

Figures 13A and 13B show the device **28** that is comprised of the PCB_2 shown in Figure 13A along with multiple electrodes 56 which have been soldered into corresponding slots **8.** It can be appreciated by one skilled in the art that the PCB **2** can be very easily and inexpensively fabricated using standard printed circuit board manufacturing processes. Further it can also be appreciated that the electrodes **56** can be easily and inexpensively attached to the slots **8** in the PCB **2** by a wave soldering station. The width of the slots **8** can be varied to accommodate different diameter electrodes and the length of the slots can be varied to ensure a long enough solder joint to secure the electrodes **56** to the PCB **2.** The slot **19** widths and lengths do not need to be the same and can be varied to accommodate different diameter and length electrodes **56** on the same PCB **2.** In this manner the entire device can be easily and reliably manufactured.

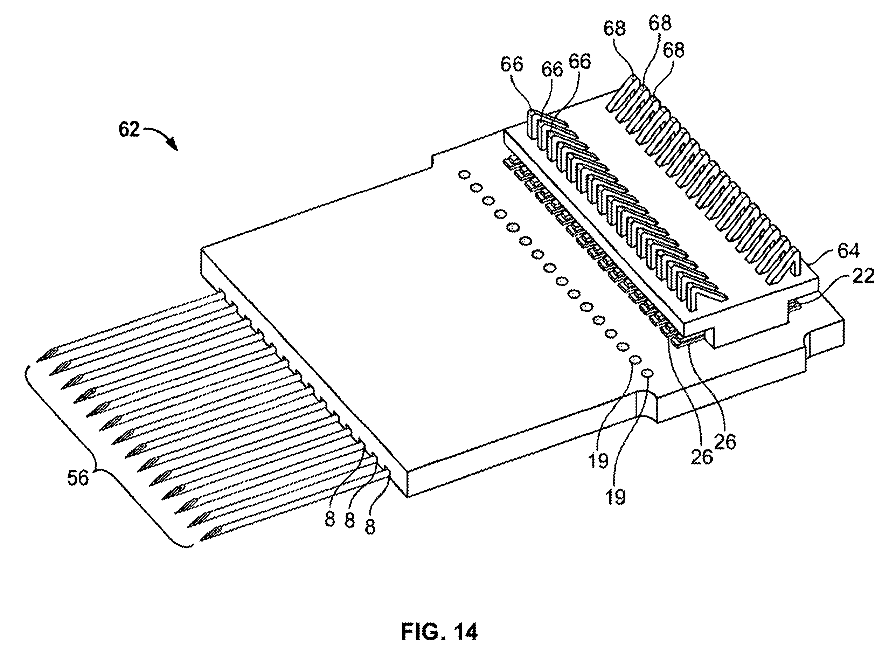

Referring now to Figure 14, an alternate device **62** is shown comprising the PCB **2** and electrodes **56** shown previously and also including an electrical connector **64.** The connector **64** has been soldered to the multiple electrical leads **26** and **22.** The connector **64** contains multiple spring leads **66** and **68** that enable connection to a carrier or delivery device. It can be appreciated that any manner of electrical connector **64** can be combined with the PCB assembly device **28** including a connector that is comprised of a cable and plug for directly connecting the PCB device **28** to a power supply (not shown).

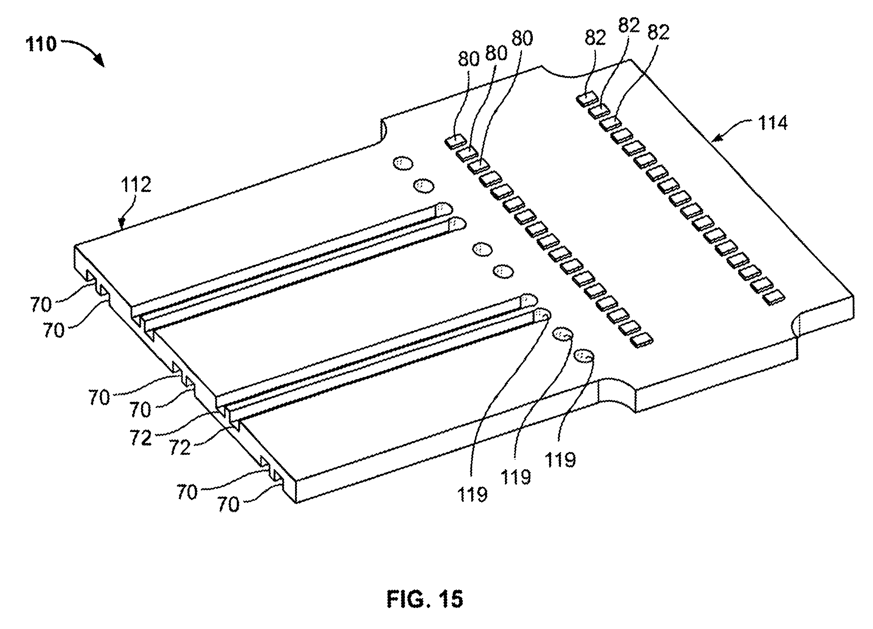

In an alternative device, shown in Figure 15, a PCB **110** is shown comprising multiple slots **70** formed in the bottom of the PCB **110** and multiple slots **72** formed in the top of the PCB **110.** All of the slots have corresponding through holes **119** which allow the multiple conductive leads in the slots **70** and **72** to be electrically connected to electrical traces (not shown) on other layers of the PCB **110.** Some of those traces are also in electrical connection to the multiple connector leads **80** and **82.** This device allows a non-linear arrangement of multiple electrodes (not shown) which may be desirable for some applications of the current device. As can also be appreciated by one skilled in the art, more than two rows of electrodes can be fabricated by utilizing additional PCBs and stacking those PCBs.

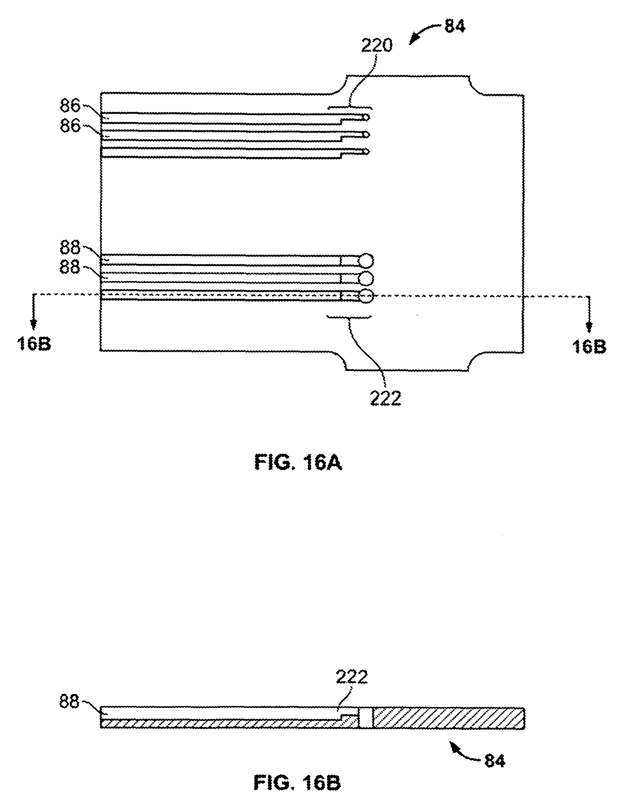

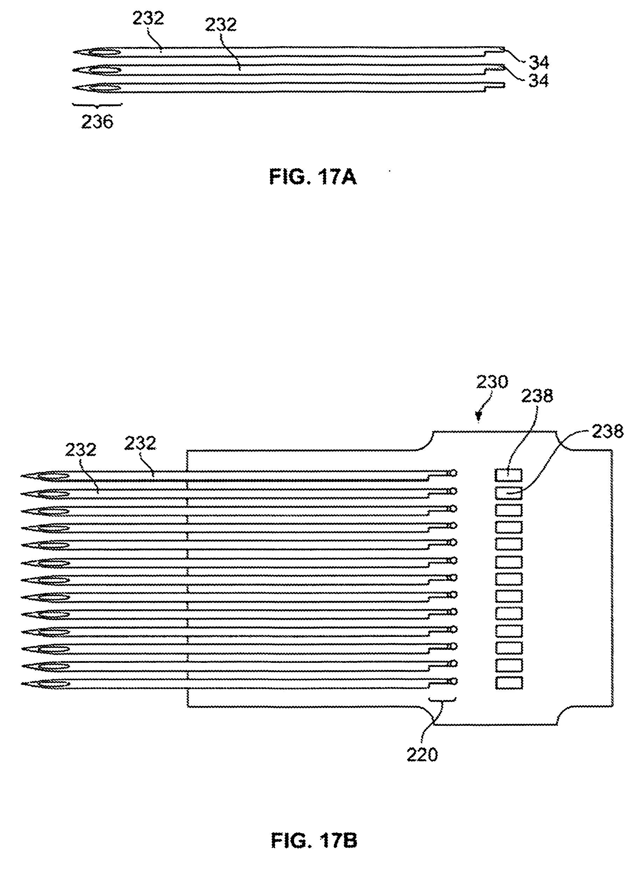

Now turning to Figures 16A and 16B, an additional benefit is shown. On this PCB **84** slots 86 have been formed that contain a narrowed proximal profile **220.** Also slots **88** have been formed which contain a shallower proximal profile **222.** Either of these alternatives can be used to rotationally locate or index the electrodes that are placed in the slots **220** and **222.** Such electrodes **232** as shown in Figure 17A can have notched features **34** at their proximal ends. These notched features **34** can correspond to a particular feature at the distal end such as the direction of the electrode bevel **236.** As shown in Figure 17B, when the notched features **34** of the electrodes **232** are fit into the narrowed proximal profile **220** on the PCB **230**, the distal bevels **236** of the electrodes **232** are aligned. This alternate version further reduces assembly time and cost by providing a method - the corresponding profile **220** or **222** and cross-sectional feature **34** - for the rotational alignment of the electrodes **232** relative to the PCB **230.**

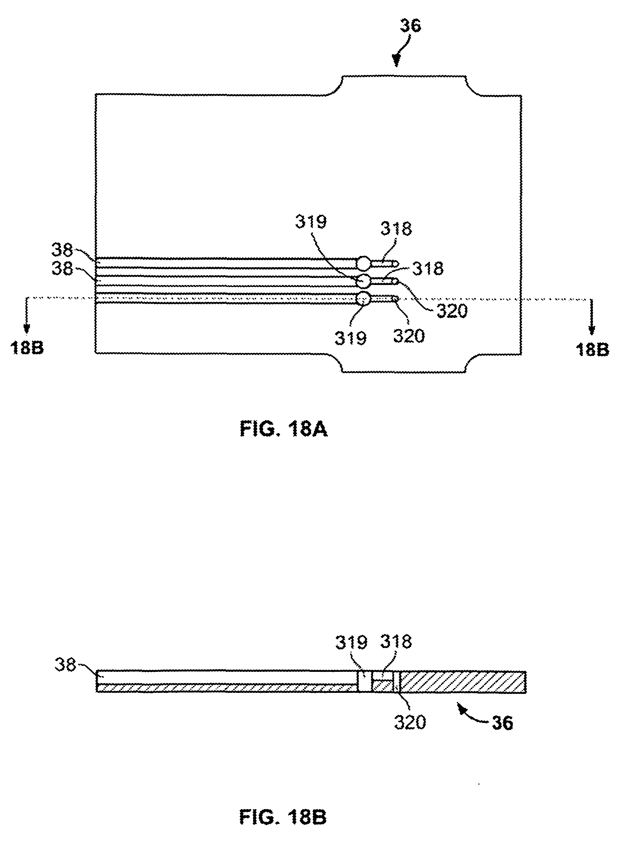

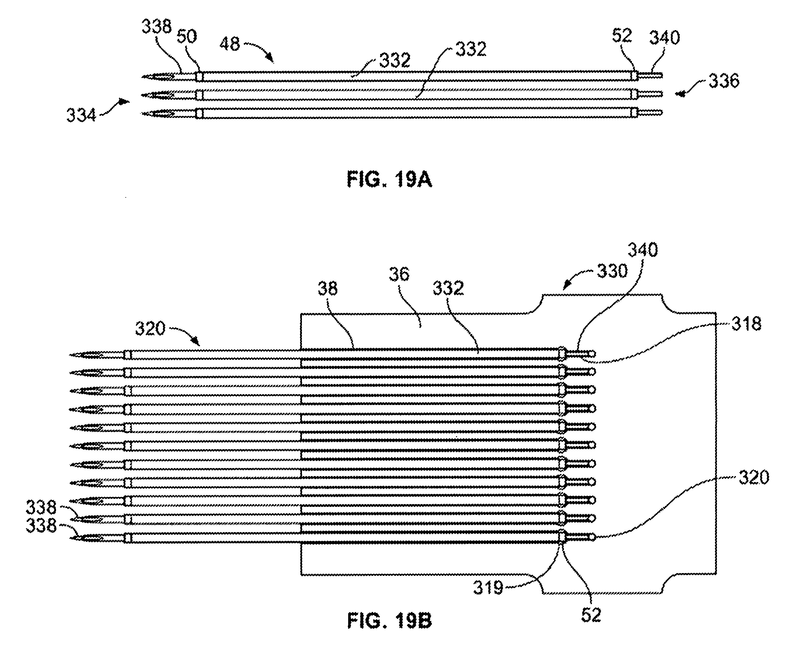

Another use of a varying slot profile is shown in Figures 18A-B and 19A-B. In Figure 18A-B a PCB **36** is shown which features electrode slots **38** which contain a smaller and shallower width proximal section **318.** This section is designed to contact a smaller diameter proximal extension **340** that is part of an electrode assembly **48.** The electrode assembly **48** is comprised of electrode cannula **332** with distal and proximal insulators **50** and **340** respectively. It also contains the distal extension **338** and the distal end **334** and the smaller diameter proximal section **318** at the proximal end **336.** The smaller diameter proximal section **318** is in electrical contact with the distal extension **338** but is electrically isolated from the electrode cannula **332.** In Figure 19B these electrodes **48** are shown attached to PCB **36** forming assembly **330.** The smaller diameter proximal section **318** is connected to the smaller and shallower width proximal section **318** of the slots **38.** The smaller and shallower width proximal section **318** is in turn in electrical contact with through hole **320** which is connected to electrical traces not shown. The electrode cannula **332** is connected to the main slot **38** which is in electrical contact to through holes **319.** The proximal insulators **52** prevent contact between the through holes **319** and the smaller and shallower width proximal section **318.** In this manner two separate electrical channels are created for each electrode assembly **48**, one from through hole **320** to distal extension **338,** and another from through hole **319** to electrode cannula **332.**

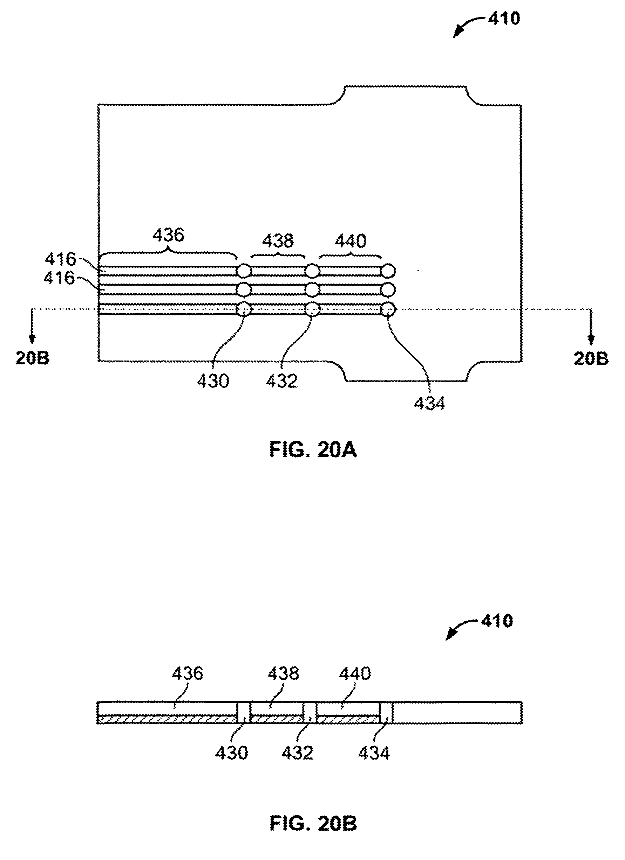

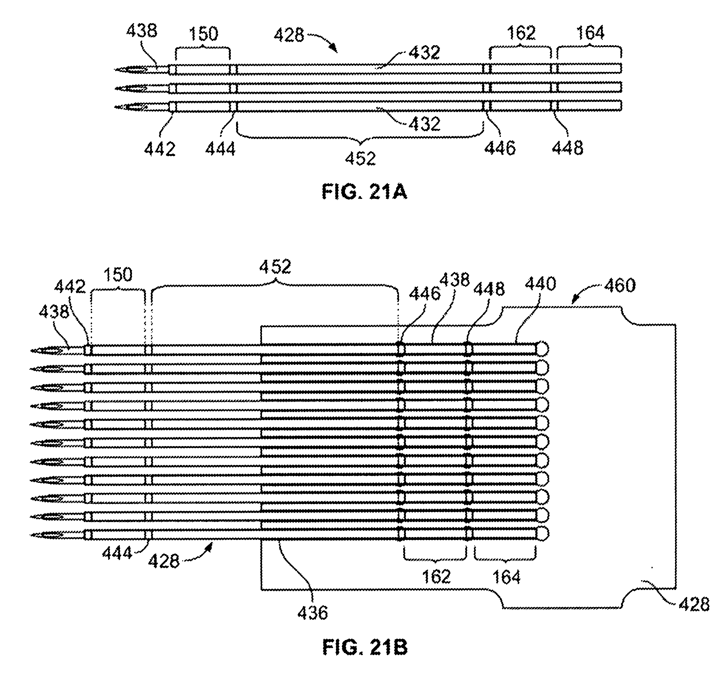

The device is not limited to just 2 independent electrical channels for each electrode assembly. It will be obvious to those skilled in the art that any number of independent channels can be created by fabricating different electrode assemblies with electrically independent sections and corresponding electrically independent slot configurations. Further the separation of slot sections to create electrically independent channels does not need to be accomplished with the use of different width or depth slots. As long as independent slot sections are provided and corresponding independent electrode sections are connected to them, any number of electrically independent channels are possible. As one example Figures 20A-B and 21A-B show a PCB device **410** with slots **416** containing three electrically independent channels **436, 438,** and **440** separated by through holes **430** and **432** respectively. These correspond to electrode assemblies **428** which have electrically independent sections **438** connected to **164, 150** connected to **162** and **432** which are separated from each other by insulators **442, 444, 446** and **448.** In this manner slots **416** which are of constant diameter are configured to connect to electrode assemblies with more than 2 independent electrical channels.

As has been described herein these independent channels can be used to deliver independent electrical currents and to read independent electrical signals.

Further the uses of various electrically independent slot features as shown in Figures 18-21 and various slot dimensions as shown in Figures 16-17 are not exclusive but can be combined in the same PCB configuration.

## Claims

1. An energy delivery device for applying energy from an energy source to treat a target region beneath a surface of tissue, the device comprising:
a device body having a handle portion, a cartridge receiving surface, and a plurality of electrically conductive leads on at least a portion of the cartridge receiving surface and being electrically coupleable to the energy source;
a cartridge body removably coupled to the device body on the cartridge receiving surface, the cartridge body comprising an electrode assembly having a plurality of electrodes arranged in an array and where at least one electrode has a connection portion **characterized in that** the device body has an actuator adjacent to the cartridge receiving surface, the actuator being moveable relative to the device body and engageable with the electrode assembly and **in that**, the electrode assembly is moveable between a treatment position and a retracted position upon movement of the actuator, such that in the treatment position the electrode extends from the cartridge body and the respective connection portion engages one electrically conductive lead, and in the retracted position, the electrode retracts into the cartridge and the respective connection portion moves out of engagement with the electrically conductive lead preventing delivery of energy.

2. The energy delivery device of claim 1, the cartridge body further comprising a tissue engaging surface, where a plane of the tissue engaging surface forms an oblique angle relative to a plane of the array of the electrodes, such that when extended from the cartridge body the electrodes extend at the oblique angle relative to the tissue engaging surface, where placement of the tissue engaging surface against tissue allows for entry of the electrodes into the tissue at the oblique angle.

3. The energy delivery device of claim 1, the plurality of electrodes comprising a first plurality of electrodes arranged in a first array, and a second plurality of electrodes arranged in a second array.

4. The energy delivery device of claim 3, where the first plurality of electrodes comprises a first length, and the second plurality of electrodes comprises a second length, where the first and second lengths are not equal such that upon insertion into tissue, each plurality of needles is adapted to extend a same vertical length into the tissue.

5. The energy delivery device of claim 3, where at least one of the first plurality of electrodes is offset from the second plurality of electrodes such that upon insertion into tissue, the plurality of electrodes does not create a continuous line of insertion points along the plurality of electrodes.

6. The energy delivery device of claim 1, the plurality of electrodes comprising a plurality of electrode pairs, where at least one electrode pair is vertically offset from an adjacent electrode pair so that insertion of electrode pairs into the tissue does not create a continuous line of insertion points.

7. The energy delivery device of claim 6, where at least one of the electrode pairs is axially offset from an adjacent electrode pair.

8. The energy delivery device of claim 1, where the actuator is coupled to a spring means and is spring-loaded and optionally the spring means is adapted to exert a spring force when restrained and when unrestrained is adapted to apply a spring force to the actuator to drive the electrode assembly from the retracted position to the treatment position with sufficient force for the electrodes to penetrate the surface of the tissue.

9. The energy delivery device of claim 1, where at least one of the electrodes comprises a temperature detecting element; and where optionally:
the plurality of electrically conductive leads comprises a first set of energy leads and a second set of temperature detecting leads, and where the energy leads and the temperature detecting leads are electrically isolated from each other; or
each connection portion of the electrode comprises an energy connection portion and a temperature detecting connection portion, and where in the treatment position, the energy connection portion contacts at least one energy lead and the temperature detecting portion contacts one temperature detecting lead, and the temperature detecting element is configured to detect a temperature of the electrode in both the retracted position and the treated position.

10. The energy delivery device of claim 1, where the device body further comprises a cooling surface removably coupled adjacent to the cartridge receiving surface such that when the electrode array is in the treatment position and the electrodes are inserted into tissue, the cooling surface is positioned to engage an area of the tissue surface directly above the electrodes, and where the cooling surface is adapted to maintain a temperature at, below, or slightly above body temperature; where optionally:
the cooling surface is visually transparent or translucent; or
the cooling surface comprises a material selected from the group consisting of steel, aluminum, and copper; or
the plurality of electrodes is adapted to pass through a portion of the cooling surface when advanced from the device body; or
the energy delivery device further comprises a thermoelectric cooling device coupled to the power supply and in contact with the cooling surface, where the thermoelectric cooling device is adapted to maintain the temperature, where the thermoelectric cooling device is optionally a Peltier cooling device; or where the device comprises
a fluid source coupled to the cooling surface, where the fluid source is adapted to maintain the temperature.

11. The energy delivery device of claim 1, where each of the electrodes has a respective impedance along a respective active region, and wherein the impedance of at least one of the electrodes varies along its respective active region, and where optionally the impedance of each of the plurality of electrodes varies along the respective active region.

12. The energy delivery device of claim 1, where the actuator is slidable or rotatable relative to the device body; or
the device body is configured to allow single handed deployment of the actuator; or
the actuator is coupled to a compressed fluid cylinder, having a valve for driving the actuator; or
a number of the conductive leads is greater than a number of the electrodes; or
the device body further comprises an ink-pad having ink and located on an exterior surface, where the ink pad is adapted to mark tissue upon contact therewith; or
the tissue engaging surface further comprises at least one sensor for detecting contact with the surface of tissue.

13. A kit for applying therapeutic energy to tissue, the kit comprising the energy delivery device of claim 1 and at least one further said cartridge body, each further said cartridge body being capable of being removably coupled to the device body on the cartridge receiving surface, and
where the electrode assembly on each respective cartridge body has a different configuration.

14. The kit of claim 13, where a number of electrodes in a first said cartridge body is different from a number of the electrodes in a second said cartridge body; or
where a length of the electrodes in a first said cartridge body is different from a length of the electrodes in a second said cartridge body; or
where a gauge of the electrodes in a first said cartridge body is different from a gauge of the electrodes in a second said cartridge body; or
where the active region of the electrodes in a first said cartridge body is different from the active region of the electrodes in a second said cartridge body; or
where the plurality of electrodes in at least a first said cartridge body comprises a first plurality of electrodes arranged in a first array, and a second plurality of electrodes arranged in a second array, where optionally at least one of the first plurality of electrodes is offset from the second plurality of electrodes such that upon insertion into tissue, the plurality of electrodes does not create a continuous line of insertion points along the plurality of electrodes; or
where the plurality of electrodes on a first cartridge body comprises a plurality of electrode pairs, where at least one electrode pair is vertically offset from an adjacent electrode pair so that insertion of electrode pairs into the tissue does not create a continuous line of insertion points; or
where a spacing between the electrodes in a first cartridge body is different from the spacing between the electrodes in a second cartridge body.

15. The energy delivery device of claim 1 or claim 2, where said actuator is a spring-loaded actuator.

## Patentansprüche

1. Energiezuführungsvorrichtung, um eine Energie von einer Energiequelle aufzubringen, um einen Zielbereich unterhalb einer Oberfläche eines Gewebes zu behandeln, wobei die Vorrichtung umfasst:
einen Vorrichtungskörper, welcher einen Griffabschnitt, eine einen Einsatz aufnehmende Fläche und mehrere elektrisch leitende Leitungen aufweist, welche auf mindestens einem Teil der einen Einsatz aufnehmenden Fläche vorhanden sind und elektrisch mit der Energiequelle koppelbar sind;
einen Einsatzkörper, welcher entfernbar mit dem Vorrichtungskörper auf der einen Einsatz aufnehmenden Fläche gekoppelt ist, wobei der Einsatzkörper eine Elektrodenanordnung umfasst, welche mehrere Elektroden aufweist, die in einer Anordnung angeordnet sind, und wobei mindestens eine Elektrode einen Verbindungsabschnitt aufweist,
**dadurch gekennzeichnet,**
**dass** der Vorrichtungskörper ein Betätigungselement benachbart zu der einen Einsatz aufnehmenden Fläche aufweist, wobei das Betätigungselement relativ zu dem Vorrichtungskörper bewegbar und mit der Elektrodenanordnung in Eingriff bringbar ist, und
**dass** die Elektrodenanordnung zwischen einer Behandlungsposition und einer zurückgezogenen Position durch eine Bewegung des Betätigungselements bewegbar ist, so dass sich die Elektroden in der Behandlungsposition von dem Einsatzkörper erstrecken und sich der entsprechende Verbindungsabschnitt mit einem elektrisch leitenden Leiter in Eingriff befindet, und so dass sich in der zurückgezogenen Position die Elektrode in den Einsatz zurückzieht und der entsprechende Verbindungsabschnitt aus einem Eingriff mit dem elektrisch leitenden Leiter bewegt, wobei eine Zuführung von Energie verhindert wird.

2. Energiezuführungsvorrichtung nach Anspruch 1, **dadurch gekennzeichnet, dass** der Einsatzkörper darüber hinaus eine Gewebeeingriffsfläche umfasst, wobei eine Ebene der Gewebeeingriffsfläche einen schiefen Winkel relativ zu einer Ebene der Anordnung der Elektroden ausbildet, so dass sich die Elektroden, wenn sie sich von dem Einsatzkörper erstrecken, in dem schiefen Winkel relativ zu der Gewebeeingriffsfläche erstrecken, wobei eine Anordnung der Gewebeeingriffsfläche gegenüber dem Gewebe ein Eindringen der Elektroden in das Gewebe in dem schiefen Winkel ermöglicht.

3. Energiezuführungsvorrichtung nach Anspruch 1, **dadurch gekennzeichnet, dass** die mehreren Elektroden eine erste Mehrzahl von Elektroden, welche in einer ersten Anordnung angeordnet sind, und eine zweite Mehrzahl von Elektroden, welche in einer zweiten Anordnung angeordnet sind, umfassen.

4. Energiezuführungsvorrichtung nach Anspruch 3, **dadurch gekennzeichnet, dass** die erste Mehrzahl von Elektroden eine erste Länge und dass die zweite Mehrzahl von Elektroden eine zweite Länge umfasst, wobei die erste und die zweite Länge nicht gleich sind, so dass bei dem Einführen in das Gewebe jede Mehrzahl von Nadeln ausgestaltet ist, um sich in einer selben vertikalen Länge in das Gewebe zu erstrecken.

5. Energiezuführungsvorrichtung nach Anspruch 3, **dadurch gekennzeichnet, dass** zumindest die erste Mehrzahl von Elektroden von der zweiten Mehrzahl von Elektroden versetzt ist, so dass beim Einführen in das Gewebe die mehreren Elektroden keine durchgängige Linie von Einführungspunkten entlang der mehreren Elektroden erzeugen.

6. Energiezuführungsvorrichtung nach Anspruch 1, **dadurch gekennzeichnet, dass** die mehreren Elektroden mehrere Elektrodenpaare umfassen, wobei mindestens ein Elektrodenpaar vertikal von einem benachbarten Elektrodenpaar versetzt ist, so dass beim Einführen der Elektrodenpaare in das Gewebe keine durchgängige Linie von Einführungspunkten erzeugt wird.

7. Energiezuführungsvorrichtung nach Anspruch 6, **dadurch gekennzeichnet, dass** mindestens eines der Elektrodenpaare axial von einem benachbarten Elektrodenpaar versetzt ist.

8. Energiezuführungsvorrichtung nach Anspruch 1, **dadurch gekennzeichnet, dass** das Betätigungselement mit Federmitteln gekoppelt ist und unter Federspannung steht und optional die Federmittel ausgestaltet sind, um eine Federkraft auszuüben, wenn sie zurückgehalten werden, und wenn sie nicht zurückgehalten werden, ausgestaltet sind, um eine Federkraft auf das Betätigungselement auszuüben, um die Elektrodenanordnung von der zurückgezogenen Position mit einer ausreichenden Kraft für die Elektroden in die Behandlungsposition anzutreiben, um die Oberfläche des Gewebes zu durchdringen.

9. Energiezuführungsvorrichtung nach Anspruch 1, **dadurch gekennzeichnet, dass** mindestens eine der Elektroden ein Temperaturerfassungselement umfasst, und wobei optional:
die mehreren elektrisch leitenden Leiter einen ersten Satz von Energieleitern und einen zweiten Satz von eine Temperatur erfassenden Leitern umfassen und wobei die Energieleiter und die eine Temperatur erfassenden Leiter elektrisch voneinander isoliert sind; oder
jeder Verbindungsabschnitt der Elektrode einen Energieverbindungsabschnitt und einen eine Temperatur erfassenden Verbindungsabschnitt umfasst und wobei in der Behandlungsposition der Energieverbindungsabschnitt mindestens einen Energieleiter berührt und der Temperatur erfassende Abschnitt einen eine Temperatur erfassenden Leiter berührt, und wobei das Temperaturerfassungselement ausgestaltet ist, um eine Temperatur der Elektrode sowohl in der zurückgezogenen Position als auch in der Behandlungsposition zu erfassen.

10. Energiezuführungsvorrichtung nach Anspruch 1, **dadurch gekennzeichnet, dass** der Vorrichtungskörper darüber hinaus eine kühlende Fläche umfasst, welche entfernbar benachbart mit der einen Einsatz aufnehmenden Fläche gekoppelt ist, so dass, wenn sich die Elektrodenanordnung in der Behandlungsposition befindet und die Elektroden in das Gewebe eingeführt sind, die kühlende Fläche angeordnet ist, um sich mit einem Bereich der Gewebeoberfläche direkt oberhalb der Elektroden in Eingriff zu befinden, und wobei die kühlende Fläche ausgestaltet ist, um eine Temperatur bei, unterhalb oder etwas oberhalb der Körpertemperatur zu halten; wobei optional:
die kühlende Fläche visuell transparent oder lichtdurchlässig ist; oder
die kühlende Fläche ein Material umfasst, welches ausgewählt ist, aus der Gruppe, bestehend aus Stahl, Aluminium und Kupfer; oder
die mehreren Elektroden ausgestaltet sind, um durch einen Abschnitt der kühlenden Fläche zu treten, wenn sie von dem Vorrichtungskörper hervortreten; oder
die Energiezuführungsvorrichtung darüber hinaus eine thermoelektrisch kühlende Vorrichtung umfasst, welche mit der Stromversorgung gekoppelt ist und sich in Kontakt mit der kühlenden Fläche befindet, wobei die thermoelektrisch kühlende Vorrichtung ausgestaltet ist, um die Temperatur zu halten, wobei die thermoelektrisch kühlende Vorrichtung optional eine Peltier-Vorrichtung ist; oder
wobei die Vorrichtung eine Fluidquelle umfasst, welche mit der kühlenden Fläche gekoppelt ist, wobei die Fluidquelle ausgestaltet ist, um die Temperatur zu halten.

11. Energiezuführungsvorrichtung nach Anspruch 1, **dadurch gekennzeichnet, dass** jede der Elektroden eine entsprechende Impedanz entlang eines entsprechenden aktiven Bereiches aufweist, und dass die Impedanz mindestens einer der Elektroden entlang ihres entsprechenden aktiven Bereichs variiert, und dass optional die Impedanz von jeder der mehreren Elektroden entlang des jeweiligen aktiven Bereiches variiert.

12. Energiezuführungsvorrichtung nach Anspruch 1, **dadurch gekennzeichnet,**
**dass** das Betätigungselement gleitbar oder drehbar relativ zu dem Vorrichtungskörper ist; oder
**dass** der Vorrichtungskörper ausgestaltet ist, um einen einhändigen Einsatz des Betätigungselements zu ermöglichen; oder
**dass** das Betätigungselement mit einem komprimierten Fluidzylinder gekoppelt ist, welcher einen Wert aufweist, um das Betätigungselement anzutreiben; oder
**dass** eine Anzahl der leitenden Leiter größer als eine Anzahl der Elektroden ist; oder dass die Vorrichtungsvorrichtung darüber hinaus ein Farbkissen umfasst, welches eine Farbe aufweist und auf einer Außenfläche angeordnet ist, wobei das Farbkissen ausgestaltet ist, um ein Gewebe bei einem Kontakt damit zu markieren; oder
wobei die Gewebeeingriffsfläche darüber hinaus mindestens einen Sensor umfasst, um einen Kontakt mit der Oberfläche des Gewebes zu erfassen.

13. Einrichtung zum Aufbringen einer therapeutischen Energie auf ein Gewebe,
wobei die Einrichtung die Energiezuführungsvorrichtung nach Anspruch 1 und darüber hinaus mindestens einen weiteren Einsatzkörper umfasst, wobei jeder weitere Einsatzkörper in der Lage ist, entfernbar mit dem Vorrichtungskörper auf der einen Einsatz aufnehmenden Fläche gekoppelt zu werden, und
wobei die Elektrodenanordnung auf jedem entsprechenden Einsatzkörper eine unterschiedliche Konfiguration aufweist.

14. Einsatz nach Anspruch 13, **dadurch gekennzeichnet, dass** eine Anzahl von Elektroden in einem ersten Einsatzkörper unterschiedlich von einer Anzahl der Elektroden in einem zweiten Einsatzkörper ist; oder
wobei eine Länge der Elektroden in einem ersten Einsatzkörper unterschiedlich von einer Länge der Elektroden in einem zweiten Einsatzkörper ist; oder
wobei eine Dicke der Elektroden in einem ersten Einsatzkörper unterschiedlich von einer Dicke der Elektroden in einem zweiten Einsatzkörper ist; oder
wobei der aktive Bereich der Elektroden in einem ersten Einsatzkörper unterschiedlich von dem aktiven Bereich der Elektroden in einem zweiten Einsatzkörper ist; oder
wobei die mehreren Elektroden in zumindest einem ersten Einsatzkörper eine erste Mehrzahl von Elektroden, welche in einer ersten Anordnung angeordnet sind, und eine zweite Mehrzahl von Elektroden, welche in einer zweiten Anordnung angeordnet sind, umfasst, wobei optional zumindest die erste Mehrzahl der Elektroden von der zweiten Mehrzahl der Elektroden versetzt ist, so dass beim Einführen in das Gewebe die mehreren Elektroden keine durchgängige Linie von Einführungspunkten entlang der mehreren Elektroden erzeugen; oder
wobei die mehreren Elektroden auf einem ersten Einsatzkörper mehrere Elektrodenpaare umfassen, wobei mindestens ein Elektrodenpaar vertikal versetzt von einem benachbarten Elektrodenpaar ist, so dass das Einführen der Elektrodenpaare in das Gewebe keine durchgängige Linie von Einführungspunkten erzeugt; oder
wobei ein Abstand zwischen den Elektroden in einem ersten Einsatzkörper unterschiedlich von einem Abstand zwischen den Elektroden in einem zweiten Einsatzkörper ist.

15. Energiezuführungsvorrichtung nach Anspruch 1 oder Anspruch 2, **dadurch gekennzeichnet, dass** das Betätigungselement ein federbelastetes Betätigungselement ist.

## Revendications

1. Dispositif de distribution d'énergie pour appliquer de l'énergie depuis une source d'énergie pour traiter une région ciblée sous une surface de tissu, le dispositif comprenant :
un corps de dispositif comportant une partie poignée, une surface de réception de cartouche et une pluralité de broches électriquement conductrices sur au moins une partie de la surface de réception de cartouche et pouvant être couplées électriquement à la source d'énergie ;
un corps de cartouche accouplé de manière amovible au corps de dispositif sur la surface de réception de cartouche, le corps de cartouche comprenant un ensemble d'électrodes comportant une pluralité d'électrodes disposées en un ensemble ordonné et où au moins une électrode a une partie de connexion, **caractérisé en ce que** le corps de dispositif comporte un actionneur adjacent à la surface de réception de cartouche, l'actionneur étant mobile par rapport au corps de dispositif et pouvant être mis en prise avec l'ensemble d'électrodes et **en ce que** l'ensemble d'électrodes est mobile entre une position de traitement et une position rétractée lors du mouvement de l'actionneur, de sorte que dans la position de traitement l'électrode s'étend depuis le corps de cartouche et la partie de connexion respective se met en prise avec une broche électriquement conductrice, et dans la position rétractée, l'électrode se rétracte dans la cartouche et la partie de connexion respective se sépare de la broche électriquement conductrice, ce qui empêche la distribution d'énergie.

2. Dispositif de distribution d'énergie selon la revendication 1, dans lequel le corps de cartouche comprend en outre une surface de contact avec le tissu, où un plan de la surface de contact avec le tissu forme un angle oblique par rapport à un plan de l'ensemble ordonné d'électrodes, de sorte que lorsqu'elles sont étendues depuis le corps de cartouche, les électrodes s'étendent selon l'angle oblique par rapport à la surface de contact avec le tissu, où la mise en place de la surface de contact avec le tissu contre le tissu permet l'entrée des électrodes dans le tissu selon l'angle oblique.

3. Dispositif de distribution d'énergie selon la revendication 1, dans lequel la pluralité d'électrodes comprend une première pluralité d'électrodes disposées en un premier ensemble ordonné, et une deuxième pluralité d'électrodes disposées en un deuxième ensemble ordonné.

4. Dispositif de distribution d'énergie selon la revendication 3, dans lequel la première pluralité d'électrodes comprend une première longueur, et la deuxième pluralité d'électrodes comprend une deuxième longueur, les première et deuxième longueurs n'étant pas égales, de sorte que lors de l'insertion dans le tissu, chaque pluralité d'aiguilles est adaptée pour s'étendre sur une même longueur verticale dans le tissu.

5. Dispositif de distribution d'énergie selon la revendication 3, dans lequel au moins une électrode de la première pluralité d'électrodes est décalée par rapport à la deuxième pluralité d'électrodes, de sorte que lors de l'insertion dans un tissu, les électrodes ne créent pas une ligne continue de points d'insertion le long de la pluralité d'électrodes.

6. Dispositif de distribution d'énergie selon la revendication 1, dans lequel la pluralité d'électrodes comprend une pluralité de paires d'électrodes, où au moins une paire d'électrodes est décalée verticalement par rapport à une paire d'électrodes adjacente, de sorte que l'insertion de paires d'électrodes dans le tissu ne crée pas une ligne continue de points d'insertion.

7. Dispositif de distribution d'énergie selon la revendication 6, dans lequel au moins l'une des paires d'électrodes est décalée axialement par rapport à une paire d'électrodes adjacente.

8. Dispositif de distribution d'énergie selon la revendication 1, dans lequel l'actionneur est accouplé à un moyen formant ressort et est à ressort et en option le moyen formant ressort est adapté pour exercer une force de ressort quand il est contraint et est adapté quand il est non contraint pour appliquer une force de ressort à l'actionneur pour entraîner l'ensemble d'électrodes de la position rétractée à la position de traitement avec une force suffisante pour que les électrodes pénètrent la surface du tissu.

9. Dispositif de distribution d'énergie selon la revendication 1, dans lequel au moins l'une des électrodes comprend un élément de détection de température, et dans lequel en option :
la pluralité de broches électriquement conductrices comprend un premier ensemble de broches d'énergie et un deuxième ensemble de broches de détection de température, et où les broches d'énergie et les broches de détection de température sont isolées électriquement les unes des autres ; ou
chaque partie de connexion de l'électrode comprend une partie de connexion d'énergie et une partie de connexion de détection de température, et où dans la position de traitement, la partie de connexion d'énergie est en contact avec au moins une broche d'énergie et la partie de détection de température est en contact avec une broche de détection de température, et l'élément de détection de température est configuré pour détecter une température de l'électrode dans la position rétractée ainsi que dans la position de traitement.

10. Dispositif de distribution d'énergie selon la revendication 1, dans lequel le corps de dispositif comprend en outre une surface de refroidissement accouplée de façon amovible en position adjacente à la surface de réception de cartouche, de sorte que lorsque l'ensemble ordonnée d'électrodes est dans la position de traitement et que les électrodes sont insérées dans un tissu, la surface de refroidissement est positionnée pour se mettre en prise avec une région de la surface du tissu située juste au-dessus des électrodes, et où la surface de refroidissement est adaptée pour maintenir une température au niveau de, sous ou légèrement au-dessus de la température corporelle ; dans lequel en option :
la surface de refroidissement est visuellement transparente ou translucide ; ou
la surface de refroidissement comprend un matériau choisi dans le groupe constitué de l'acier, l'aluminium et le cuivre ; ou
la pluralité d'électrodes est adaptée pour passer à travers une partie de la surface de refroidissement quand on la fait avancer depuis le corps de dispositif ; ou
le dispositif de distribution d'énergie comprend en outre un dispositif de refroidissement thermoélectrique couplé à l'alimentation électrique et en contact avec la surface de refroidissement, dans lequel le dispositif de refroidissement thermoélectrique est adapté pour maintenir la température, le dispositif de refroidissement thermoélectrique étant en option un dispositif de refroidissement de Peltier ; ou dans lequel le dispositif comprend
une source de fluide couplée à la surface de refroidissement, la source de fluide étant adaptée pour maintenir la température.

11. Dispositif de distribution d'énergie selon la revendication 1, dans lequel chacune des électrodes a une impédance respective le long d'une région active respective, et dans lequel l'impédance d'au moins l'une des électrodes varie le long de sa région active respective, et où en option l'impédance de chacune des électrodes varie le long de la région active respective.

12. Dispositif de distribution d'énergie selon la revendication 1, dans lequel l'actionneur peut glisser ou tourner par rapport au corps de dispositif ; ou
le corps de dispositif est configuré pour permettre un déploiement d'une main de l'actionneur ; ou
l'actionneur est couplé à un vérin à fluide comprimé, comportant une valve pour entraîner l'actionneur ; ou
le nombre des broches conductrices est supérieur au nombre des électrodes ; ou
le corps de dispositif comprend en outre un tampon à encre comportant de l'encre et situé sur une surface extérieure, le tampon à encre étant adapté pour marquer le tissu lors du contact avec celui-ci ; ou
la surface de contact avec le tissu comprend en outre au moins un capteur pour détecter le contact avec la surface de tissu.

13. Nécessaire pour l'application d'énergie thérapeutique à un tissu, le nécessaire comprenant le dispositif de distribution d'énergie de la revendication 1 et au moins un autre dit corps de cartouche, chaque dit autre corps de cartouche étant apte à être accouplé de façon amovible au corps de dispositif sur la surface de réception de cartouche, et
dans lequel l'ensemble d'électrodes sur chaque corps de cartouche respectif a une configuration différente.

14. Nécessaire selon la revendication 13, dans lequel un nombre d'électrodes dans un premier dit corps de cartouche est différent d'un nombre d'électrodes dans un deuxième dit corps de cartouche ; ou
dans lequel une longueur des électrodes dans un premier dit corps de cartouche est différente d'une longueur des électrodes dans un deuxième dit corps de cartouche ; ou
dans lequel un calibre des électrodes dans un premier dit corps de cartouche est différent d'un calibre des électrodes dans un deuxième dit corps de cartouche ; ou
dans lequel la région active des électrodes dans un premier dit corps de cartouche est différente de la région active des électrodes dans un deuxième dit corps de cartouche ; ou
dans lequel la pluralité d'électrodes dans au moins un premier dit corps de cartouche comprend une première pluralité d'électrodes disposées en un premier ensemble ordonné, et une deuxième pluralité d'électrodes disposées en un deuxième ensemble ordonné, dans lequel en option au moins l'une des premières électrodes est décalée par rapport à la deuxième pluralité d'électrodes, de sorte que lors de l'insertion dans un tissu, la pluralité d'électrodes ne crée pas une ligne continue de points d'insertion le long de la pluralité d'électrodes ; ou
dans lequel la pluralité d'électrodes d'un premier corps de cartouche comprend une pluralité de paires d'électrodes, où au moins une paire d'électrodes est décalée verticalement par rapport à une paire d'électrodes adjacente, de sorte que l'insertion de paires d'électrodes dans le tissu ne crée pas une ligne continue de points d'insertion ; ou
dans lequel un espacement entre les électrodes d'un premier corps de cartouche est différent de l'espacement entre les électrodes d'un deuxième corps de cartouche.

15. Dispositif de distribution d'énergie selon la revendication 1 ou 2, dans lequel ledit actionneur est un actionneur à ressort.
